# EUROPEAN PATENT SPECIFICATION

(11) **EP 2 152 081 B1**
(45) Date of publication and mention of the grant of the patent: **24.10.2012**
(21) Application number: 08745333.8
(22) Date of filing: 09.04.2008
(51) Int. Cl.: A01N 43/64, A61K 31/53

(54) **NOVEL COMPOUNDS**
NEUE VERBINDUNGEN
NOUVEAUX COMPOSÉS

(30) Priority: 11.04.2007 EP 07105938; 13.04.2007 US 923251 P
(43) Date of publication of application: 17.02.2010
(73) Proprietor: High Point Pharmaceuticals, LLC, High Point, NC 27265 (US)
(72) Inventor: PETERSEN, Anders Klarskov, DK-2850 Naerum (DK); EBDRUP, Soren, DK-4000 Roskilde (DK)
(74) Representative: Gillard, Richard Edward
(86) International application number: PCT/US2008/059700
(87) International publication number: WO 2008/127924

(56) References cited:
- WO-A2-2004/089470
- US-A1- 2004 224 996
- US-A1- 2005 261 302
- US-A1- 2006 009 918
- US-A1- 2006 079 506
- US-A1- 2006 111 348

## Description

The present invention relates to novel substituted benzamide based inhibitors, to their use in therapy, to pharmaceutical compositions comprising the compounds, to the use of said compounds in the manufacture of medicaments, and to therapeutic methods comprising the administration of said compounds. The present compounds modulate the activity of 11β-hydroxysteroid dehydrogenase type 1 (11βHSD1) and are accordingly useful in the treatment of diseases in which such a modulation is beneficial, such as the metabolic syndrome.

### BACKGROUND OF THE INVENTION

The metabolic syndrome is a major global health problem. In the US, the prevalence in the adult population is currently estimated to be approximately 25%, and it continues to increase both in the US and worldwide. The metabolic syndrome is characterised by a combination of insulin resistance, dyslipidemia, obesity and hypertension leading to increased morbidity and mortality of cardiovascular diseases. People with the metabolic syndrome are at increased risk of developing frank type 2 diabetes, the prevalence of which is equally escalating.

In type 2 diabetes, obesity and dyslipidemia are also highly prevalent and around 70% of people with type 2 diabetes additionally have hypertension once again leading to increased mortality of cardiovascular diseases.

In the clinical setting, it has long been known that glucocorticoids are able to induce all of the cardinal features of the metabolic syndrome and type 2 diabetes.

11β-hydroxysteroid dehydrogenase type 1 (11βHSD1) catalyses the local generation of active glucocorticoid in several tissues and organs including predominantly the liver and adipose tissue, but also e.g. skeletal muscle, bone, pancreas, endothelium, ocular tissue and certain parts of the central nervous system. Thus, 11βHSD1 serves as a local regulator of glucocorticoid actions in the tissues and organs where it is expressed (Tannin et al., J. Biol. Chem., 266, 16653 (1991); Bujalska et al., Endocrinology, 140, 3188 (1999); Whorwood et al., J Clin Endocrinol Metab., 86, 2296 (2001); Cooper et al., Bone, 27, 375 (2000); Davani et al., J. Biol. Chem., 275, 34841 (2000); Brem et al., Hypertension, 31, 459 (1998); Rauz et al., Invest. Ophthalmol. Vis. Sci., 42, 2037 (2001); Moisan et al., Endocrinology, 127, 1450 (1990)).

The role of 11βHSD1 in the metabolic syndrome and type 2 diabetes is supported by several lines of evidence. In humans, treatment with the non-specific 11βHSD1 inhibitor carbenoxolone improves insulin sensitivity in lean healthy volunteers and people with type 2 diabetes. Likewise, 11βHSD1 knock-out mice are resistant to insulin resistance induced by obesity and stress. Additionally, the knock-out mice present with an anti-atherogenic lipid profile of decreased VLDL triglycerides and increased HDL-cholesterol. Conversely, mice that over-express 11βHSD1 in adipocytes develop insulin resistance, hyperlipidemia and visceral obesity, a phenotype that resembles the human metabolic syndrome (Andrews et al., J. Clin. Endocrinol. Metab., 88, 285 (2003); Walker et al., J. Clin. Endocrinol. Metab., 80, 3155 (1995); Morton et al., J. Biol. Chem., 276, 41293 (2001); Kotelevtsev et al., Proc. Natl. Acad. Sci. USA, 94, 14924 (1997); Masuzaki et al., Science, 294, 2166 (2001)).

The more mechanistic aspects of 11βHSD1 modulation and thereby modulation of intracellular levels of active glucocorticoid have been investigated in several rodent models and different cellular systems. 11βHSD1 promotes the features of the metabolic syndrome by increasing hepatic expression of the rate-limiting enzymes in gluconeogenesis, namely phos-phoenolpyuvate carboxykinase and glucose-6-phosphatase, promoting the differentiation of preadipocytes into adipocytes thus facilitating obesity, directly and indirectly stimulating hepatic VLDL secretion, decreasing hepatic LDL uptake and increasing vessel contractility (Kotelevtsev et al., Proc. Natl. Acad. Sci. USA, 94, 14924(1997); Morton et al., J. Biol. Chem. 276, 41293 (2001); Bujalska et al., Endocrinology, 140, 3188 (1999); Souness et al., Steroids, 67, 195 (2002), Brindley & Salter, Prog. Lipid Res., 30, 349 (1991)).

WO 01/90090, WO 01/90091, WO 01/90092, WO 01/90093 and WO 01/90094 discloses various thiazol-sulfonamides as inhibitors of the human 11β-hydroxysteroid dehydrogenase type 1 enzyme, and further states that said compounds may be useful in treating diabetes, obesity, glaucoma, osteoporosis, cognitive disorders, immune disorders and depression. WO 2004/089470 discloses various substituted amides and the use thereof for stimulating 11β-hydroxysteroid dehydrogenase type 1. WO 2004/089415 and WO 2004/089416 disclose various combination therapies using an 11β-hydroxysteroid dehydrogenase type 1 inhibitor and respectively a glucocorticoid receptor agonist or an antihypertensive agent.

We have now found new substituted benzamide based inhibitors that modulate the activity of 11βHSD1 leading to altered intracellular concentrations of active glucocorticoid. More specifically, the present compounds inhibit the activity of 11βHSD1 leading to decreased intracellular concentrations of active glucocorticoid. Thus, the present compounds can be used to treat disorders where a decreased level of active intracellular glucocorticoid is desirable, such as e.g. the metabolic syndrome, type 2 diabetes, impaired glucose tolerance (IGT), impaired fasting glucose (IFG), dyslipidemia, obesity, hypertension, diabetic late complications, cardiovascular diseases, arteriosclerosis, atherosclerosis, myopathy, muscle wasting, osteoporosis, neurodegenerative and psychiatric disorders, and adverse effects of treatment or therapy with glucocorticoid receptor agonists.

One object of the present invention is to provide compounds, pharmaceutical compositions and use of compounds that modulate the activity of 11βHSD1.

### DEFINITIONS

The term "halogen" or "halo" means fluorine, chlorine, bromine or iodine.

The term "hydroxy" shall mean the radical -OH.

The term "carboxy" shall mean the radical -(C=O)OH.

The term "cyano" shall mean the radical -CN.

The term "C₁-C₆alkyl" as used herein represents a saturated, branched or straight hydrocarbon group having from 1 to 6 carbon atoms, e.g. C₁-C₂alkyl, C₁-C₃alkyl, C₁-C₋₄alkyl, C₁-C₆alkyl, C₂-C₆alkyl, C₃-C₆alkyl, and the like. Representative examples are methyl, ethyl, propyl (e.g. prop-1-yl, prop-2-yl (or iso-propyl)), butyl (e.g. 2-methylprop-2-yl (or *tert-*butyl), but-1-yl, but-2-yl), pentyl (e.g. pent-1-yl, pent-2-yl, pent-3-yl), 2-methylbut-1-yl, 3-methylbut-1-yl, hexyl (e.g. hex-1-yl), and the like. The term "C₁-C₄alkyl" as used herein represents a saturated, branched or straight hydrocarbon group having from 1 to 4 carbon atoms, e.g. C₁-C₂alkyl, C₁-C₃alkyl, C₁-C₋₄alkyl, and the like. Representative examples are methyl, ethyl, propyl (e.g. prop-1-yl, prop-2-yl (or iso-propyl)), butyl (e.g. 2-methylprop-2-yl (or tert-butyl), but-1-yl, but-2-yl), and the like.

The term "bridge" as used herein represents a connection in a saturated or partly saturated ring between two atoms of such ring that are not neighbors through a chain of 1 to 3 atoms selected from carbon, nitrogen, oxygen and sulfur. Representative examples of such connecting chains are -CH₂-, -CH₂CH₂-, -CH₂NHCH₂-, -CH₂CH₂CH₂-, -CH₂OCH₂-, and the like. In one embodiment according to the invention, the connecting chain is selected from the group consisting of -CH₂-, -CH₂CH₂-, or -CH₂OCH₂-.

The term "spiro atom" as used herein represents a carbon atom in a saturated or partly saturated ring that connects both ends of a chain of 3 to 7 atoms selected from carbon, nitrogen, oxygen and sulfur. Representative examples are -(CH₂)₅-, -(CH₂)₃-, -(CH₂)₄-, - CH₂NHCH₂CH₂-, -CH₂CH₂NHCH₂CH₂-, -CH₂NHCH₂CH₂CH₂-, -CH₂CH₂OCH₂-, -OCH₂CH₂O-, and the like.

The term "C₃-C₁₀cycloalkyl" as used herein represents a saturated monocyclic carbocyclic ring having from 3 to 10 carbon atoms, e.g. C₃₋₆-alkyl, C₃₋₈-alkyl, C₃₋₁₀-alkyl, and the like. In one aspect of the invention, C₃-C₁₀cycloalkyl is C₃-C₆cycloalkyl. Representative examples of C₃-C₁₀cycloalkyl are cyclopropyl, cyclobutyl, cyclopentyl, cyclohexyl, cycloheptyl, cyclooctyl, and the like. Representative examples of C₃-C₆cycloalkyl are cyclopropyl, cyclobutyl, cyclopentyl, and cyclohexyl. C₃-C₁₀cycloalkyl is also intended to represent a saturated bicyclic carbocyclic ring having from 4 to 10 carbon atoms. Representative examples are decahydronaphthalenyl, bicyclo[3.3.0]octanyl, and the like. C₃-C₁₀cycloalkyl is also intended to represent a saturated carbocyclic ring having from 3 to 10 carbon atoms and containing one or two carbon bridges. Representative examples are adamantyl, norbornanyl, nortricyclyl, bicyclo[3.2.1]octanyl, bicyclo[2.2.2]octanyl, tricyclo[5.2.1.0/2,6]decanyl, bicyclo[2.2.1]heptyl, and the like. C₃-C₁₀cycloalkyl is also intended to represent a saturated carbocyclic ring having from 3 to 10 carbon atoms and containing one or more spiro atoms. Representative examples are spiro[2.5]octanyl, spiro[4.5]decanyl, and the like.

The term "C₃-C₁₀heterocyclyl" as used herein represents a saturated 3 to 10 membered monocyclic ring, containing one or more heteroatoms selected from nitrogen, oxygen, sulfur, S(=O) and S(=O)₂. In a preferred embodiment, C₃-C₁₀heterocyclyl is C₃-C₆heterocyclyl. Representative examples of C₃-C₁₀heterocyclyl are aziridinyl (e.g. aziridin-1-yl), azetidinyl (e.g. azetidin-1-yl, azetidin-3-yl), oxetanyl, pyrrolidinyl (e.g. pyrrolidin-1-yl, pyrrolidin-2-yl, pyrrolidin-3-yl), imidazolidinyl (e.g. imidazolidin-1-yl, imidazolidin-2-yl, imidazolidin-4-yl), oxazolidinyl (e.g. oxazolidin-2-yl, oxazolidin-3-yl, oxazolidin-4-yl), thiazolidinyl (e.g. thiazolidin-2-yl, thiazolidin-3-yl, thiazolidin-4-yl), isothiazolidinyl, piperidinyl (e.g. piperidin-1-yl, piperidin-2-yl, piperidin-3-yl, piperidin-4-yl), homopiperidinyl (e.g. homopiperidin-1-yl, homopiperidin-2-yl, homopiperidin-3-yl, homopiperidin-4-yl), piperazinyl (e.g. piperazin-1-yl, piperazin-2-yl), morpholinyl (e.g. morpholin-2-yl, morpholin-3-yl, morpholin-4-yl), thiomorpholinyl (e.g. thiomorpholin-2-yl, thiomorpholin-3-yl, thiomorpholin-4-yl), 1-oxo-thiomorpholinyl, 1,1-dioxo-thiomorpholinyl, tetrahydrofuranyl (e.g. tetrahydrofuran-2-yl, tetrahydrofuran-3-yl), tetrahydrothienyl, tetrahydro-1,1-dioxothienyl, tetrahydropyranyl (e.g. 2-tetrahydropyranyl), tetrahydrothiopyranyl (e.g. 2-tetrahydrothiopyranyl), 1,4-dioxanyl, 1,3-dioxanyl, and the like. C₃-C₁₀heterocyclyl is also intended to represent a saturated 6 to 10 membered bicyclic ring containing one or more heteroatoms selected from nitrogen, oxygen, sulfur, S(=O) and S(=O)₂. Representative examples are octahydroindolyl (e.g. octahydroindol-1-yl, octahydroindol-2-yl, octahydroindol-3-yl, octahydroindol-5-yl), decahydroquinolinyl (e.g. decahydroquinolin-1-yl, decahydroquinolin-2-yl, decahydroquinolin-3-yl, decahydroquinolin-4-yl, decahydroquinolin-6-yl), decahydroquinoxalinyl (e.g. decahydroquinoxalin-1-yl, decahydroquinoxalin-2-yl, decahydroquinoxalin-6-yl) and the like. C₃-C₁₀-heterocyclyl is also intended to represent a saturated 6 to 10 membered ring containing one or more heteroatoms selected from nitrogen, oxygen, sulfur, S(=O) and S(=O)₂ and having one or two bridges. Representative examples are 3-azabicyclo[3.2.2]nonyl, 2-azabicycle-[2.2.1]heptyl, 3-azabicyclo[3.1.0]hexyl, 2,5-diazabicyclo[2.2.1]heptyl, atropinyl, tropinyl, quinuclidinyl, 1,4-diazabicyclo[2.2.2]octanyl, and the like. C₃-C₁₀heterocyclyl is also intended to represent a 6 to 10 membered saturated ring containing one or more heteroatoms selected from nitrogen, oxygen, sulfur, S(=O) and S(=O)₂ and containing one or more spiro atoms. Representative examples are 1,4-dioxaspiro[4.5]decanyl (e.g. 1,4-dioxa-spiro[4.5]decan-2-yl, 1,4-dioxaspiro[4.5]decan-7-yl), 1,4-dioxa-8-azaspiro[4.5]decanyl (e.g. 1,4-dioxa-8-azaspiro[4.5]decan-2-yl, 1,4-dioxa-8-azaspiro[4.5]decan-8-yl), 8-azaspiro[4.5]-decanyl (e.g. 8-azaspiro[4.5]decan-1-yl, 8-azaspiro[4.5]decan-8-yl), 2-azaspiro[5.5]unde-canyl (e.g. 2-azaspiro[5.5]undecan-2-yl), 2,8-diazaspiro[4.5]decanyl (e.g. 2,8-diazaspiro-[4.5]decan-2-yl, 2,8-diazaspiro[4.5]decan-8-yl), 2,8-diazaspiro[5.5]undecanyl (e.g. 2,8-diazaspiro[5.5]undecan-2-yl), 1,3,8-triazaspiro[4.5]decanyl (e.g. 1,3,8-triazaspiro[4.5]-decan-1-yl, 1,3,8-triazaspiro[4.5]decan-3-yl, 1,3,8-triazaspiro[4.5]decan-8-yl), and the like.

The term "heteroaryl" as used herein is intended to include monocyclic heterocyclic aromatic rings containing one or more heteroatoms selected from nitrogen, oxygen, sulfur, SO and S(=O)₂. Representative examples are pyrrolyl (e.g. pyrrol-1-yl, pyrrol-2-yl, pyrrol-3-yl), furanyl (e.g. furan-2-yl, furan-3-yl), thienyl (e.g. thien-2-yl, thien-3-yl), oxazolyl (e.g. oxazol-2-yl, oxazol-4-yl, oxazol-5-yl), thiazolyl (e.g. thiazol-2-yl, thiazol-4-yl, thiazol-5-yl), imidazolyl (e.g. imidazol-2-yl, imidazol-4-yl, imidazol-5-yl), pyrazolyl (e.g. pyrazol-1-yl, pyrazol-3-yl, pyrazol-5-yl), isoxazolyl (e.g. isoxazol-3-yl, isoxazol-4-yl, isoxazol-5-yl), isothiazolyl (e.g. isothiazol-3-yl, isothiazol-4-yl, isothiazol-5-yl), 1,2,3-triazolyl (e.g. 1,2,3-triazol-1-yl, 1,2,3-triazol-4-yl, 1,2,3-triazol-5-yl), 1,2,4-triazolyl (e.g. 1,2,4-triazol-1-yl, l,2,4-triazol-3-yl, 1,2,4-triazol-5-yl), 1,2,3-oxadiazolyl (e.g. 1,2,3-oxadiazol-4-yi, 1,2,3-oxadiazol-5-yl), 1,2,4-oxadiazolyl (e.g. 1,2,4-oxadiazol-3-yl, 1,2,4-oxadiazol-5-yi), 1,2,5-oxadiazolyl (e.g. 1,2,5-oxadiazol-3-yl, 1,2,5-oxadiazol-4-yl), 1,3,4-oxadiazolyl (e.g. 1,3,4-oxadiazol-2-yl, 1,3,4-oxadiazol-5-yi), 1,2,3-thiadiazolyl (e.g. 1,2,3-thiadiazol-4-yl, 1,2,3-thiadiazol-5-yl), 1,2,4-thiadiazolyl (e.g. 1,2,4-thiadiazol-3-yl, 1,2,4-thiadiazol-5-yi), 1,2,5-thiadiazolyl (e.g. 1,2,5-thiadiazol-3-yl, 1,2,5-thiadiazol-4-yl), 1,3,4-thiadiazolyl (e.g. 1,3,4-thiadiazol-2-yl, 1,3,4-thiadiazol-5-yl), tetrazolyl (e.g. tetrazol-1-yl, tetrazol-5-yl), pyranyl (e.g. pyran-2-yl), pyridinyl (e.g. pyridine-2-yl, pyridine-3-yl, pyridine-4-yl), pyridazinyl (e.g. pyridazin-2-yl, pyridazin-3-yl), pyrimidinyl (e.g. pyrimidin-2-yl, pyrimidin-4-yl, pyrimidin-5-yl), pyrazinyl, 1,2,3-triazinyl, 1,2,4-triazinyl, 1,3,5-triazinyl, thiadiazinyl, azepinyl, azecinyl, and the like. Heteroaryl is also intended to include bicyclic heterocyclic aromatic rings containing one or more heteroatoms selected from nitrogen, oxygen, sulfur, S(=O) and S(=O)₂. Representative examples are indolyl (e.g. indol-1-yl, indol-2-yl, indol-3-yl, indol-5-yl), isoindolyl, benzofuranyl (e.g. benzo[b]furan-2-yl, benzo[b]furan-3-yl, benzo[b]furan-5-yl, benzo[c]furan-2-yl, benzo[c]furan-3-yl, benzo[c]furan-5-yl), benzothienyl (e.g. benzo-[b]thien-2-yl, benzo[b]thien-3-yl, benzo[b]thien-5-yl, benzo[c]thien-2-yl, benzo[c]thien-3-yl, benzo[c]thien-5-yl), indazolyl (e.g. indazol-1-yl, indazol-3-yl, indazol-5-yl), indolizinyl (e.g. indolizin-1-yl, indolizin-3-yl), benzopyranyl (e.g. benzo[b]pyran-3-yl, benzo[b]pyran-6-yl, benzo[c]pyran-1-yl, benzo[c]pyran-7-yl), benzimidazolyl (e.g. benzimidazol-1-yl, benzimidazol-2-yl, benzimidazol-5-yl), benzothiazolyl (e.g. benzothiazol-2-yl, benzothiazol-5-yl), benzisothiazolyl, benzoxazolyl, benzisoxazolyl, benzoxazinyl, benzotriazolyl, naphthyridinyl (e.g. 1,8-naphthyridin-2-yl, 1,7-naphthyridin-2-yl, 1,6-naphthyridin-2-yl), phthalazinyl (e.g. phthalazin-1-yl, phthalazin-5-yl), pteridinyl, purinyl (e.g. purin-2-yl, purin-6-yl, purin-7-yl, purin-8-yl, purin-9-yl), quinazolinyl (e.g. quinazolin-2-yl, quinazolin-4-yl, quinazolin-6-yl), cinnolinyl, quinoliny (e.g. quinolin-2-yl, quinolin-3-yl, quinolin-4-yl, quinolin-6-yl), isoquinolinyl (e.g. isoquinolin-1-yl, isoquinolin-3-yl, isoquinolin-4-yl), quinoxalinyl (e.g. quinoxalin-2-yl, quinoxalin-5-yl), pyrrolopyridinyl (e.g. pyrrolo[2,3-b]pyridinyl, pyrrolo[2,3-c]pyridinyl, pyrrolo[3,2-c]pyridinyl), furopyridinyl (e.g. furo[2,3-b]pyridinyl, furo[2,3-c]pyridinyl, furo-[3,2-c]pyridinyl), thienopyridinyl (e.g. thieno[2,3-b]pyridinyl, thieno[2,3-c]pyridinyl, thieno-[3,2-c]pyridinyl), imidazopyridinyl (e.g. imidazo[4,5-b]pyridinyl, imidazo[4,5-c]pyridinyl, imidazo[1,5-a]pyridinyl, imidazo[1,2-a]pyridinyl), imidazopyrimidinyl (e.g. imidazo[1,2-a]pyrimidinyl, imidazo[3,4-a]pyrimidinyl), pyrazolopyridinyl (e.g. pyrazolo[3,4-b]pyridinyl, pyrazolo[3,4-c]pyridinyl, pyrazolo[1,5-a]pyridinyl), pyrazolopyrimidinyl (e.g. pyrazolo[1,5-a]pyrimidinyl, pyrazolo[3,4-d]pyrimidinyl), thiazolopyridinyl (e.g. thiazolo[3,2-d]pyridinyl), thiazolopyrimidinyl (e.g. thiazolo[5,4-d]pyrimidinyl), imdazothiazolyl (e.g. imidazo[2,1-b]-thiazolyl), triazolopyridinyl (e.g. triazolo[4,5-b]pyridinyl), triazolopyrimidinyl (e.g. 8-aza-purinyl), and the like. Heteroaryl is also intended to include polycyclic heterocyclic aromatic rings containing one or more heteroatoms selected from nitrogen, oxygen, sulfur, S(=O) and S(=O)₂. Representative examples are carbazolyl (e.g. carbazol-2-yl, carbazol-3-yl, carbazol-9-yl), phenoxazinyl (e.g. phenoxazin-10-yl), phenazinyl (e.g. phenazin-5-yl), acridinyl (e.g. acridin-9-yl, acridin-10-yl), phenothiazinyl (e.g. phenothiazin-10-yl), carbolinyl (e.g. pyrido-[3,4-b]indol-1-yl, pyrido[3,4-b]indol-3-yl), phenanthrolinyl (e.g. phenanthrolin-5-yl), and the like. Heteroaryl is also intended to include partially saturated monocyclic, bicyclic or polycyclic heterocyclic rings containing one or more heteroatoms selected from nitrogen, oxygen, sulfur, S(=O) and S(=O)₂. Representative examples are pyrrolinyl, pyrazolinyl, imidazolinyl (e.g. 4,5-dihydroimidazol-2-yl, 4,5-dihydroimidazol-1-yl), indolinyl (e.g. 2,3-dihydroindol-1-yl, 2,3-dihydroindol-5-yl), dihydrobenzofuranyl (e.g. 2,3-dihydrobenzo[b]furan-2-yl, 2,3-dihydrobenzo[b]furan-4-yl), dihydrobenzothienyl (e.g. 2,3-dihydrobenzo[b]thien-2-yl, 2,3-dihydrobenzo[b]thien-5-yl), 4,5,6,7-tetrahydrobenzo[b]furan-5-yl), dihydrobenzopyranyl (e.g. 3,4-dihydrobenzo[b]pyran-3-yl, 3,4-dihydrobenzo[b]pyran-6-yl, 3,4-dihydrobenzo-[c]pyran-1-yl, dihydrobenzo[c]pyran-7-yl), oxazolinyl (e.g. 4,5-dihydrooxazol-2-yl, 4,5-dihydrooxazol-4-yl, 4,5-dihydrooxazol-5-yl), isoxazolinyl, oxazepinyl, tetrahydroindazolyl (e.g. 4,5,6,7-tetrahydroindazol-1-yl, 4,5,6,7-tetrahydroindazol-3-yl, 4,5,6,7-tetrahydroindazol-4-yl, 4,5,6,7-tetrahydroindazol-6-yl), tetrahydrobenzimidazolyl (e.g. 4,5,6,7-tetrahydrobenzimidazol-1-yl, 4,5,6,7-tetrahydrobenzimidazol-5-yl), tetrahydroimidazo[4,5-c]-pyridyl (e.g. 4,5,6,7-tetrahydroimidazo[4,5-c]pyrid-1-yl, 4,5,6,7-tetrahydroimidazo[4,5-c]-pyrid-5-yl, 4,5,6,7-tetrahydroimidazo[4,5-c]pyrid-6-yl), tetrahydroquinolinyl (e.g. 1,2,3,4-tetrahydroquinolinyl, 5,6,7,8-tetrahydroquinolinyl), tetrahydroisoquinolinyl (e.g. 1,2,3,4-tetrahydroisoquinolinyl, 5,6,7,8-tetrahydroisoquinolinyl), tetrahydroquinoxalinyl (e.g. 1,2,3,4-tetrahydroquinoxalinyl, 5,6,7,8-tetrahydroquinoxalinyl), and the like. Heteroaryl is also intended to include partially saturated bicyclic or polycyclic heterocyclic rings containing one or more spiro atoms. Representative examples are spiro[isoquinoline-3,1'-cyclohexan]-1-yl, spiro[piperidine-4,1'-benzo[c]thiophen]-1-yl, spiro[piperidine-4,1'-benzo[c]furan]-1-yl, spiro[piperidine-4,3'-benzo[b]furan]-1-yl, spiro[piperidine-4,3'-coumarin]-1-yl, and the like.

The term "monocyclic heteroaryl" as used herein is intended to include monocyclic heterocyclic aromatic rings as defined above.

The term "bicyclic heteroaryl" as used herein is intended to include bicyclic heterocyclic aromatic rings as defined above.

Certain of the defined terms may occur more than once in the structural formulae, and upon such occurrence each term shall be defined independently of the other.

Certain of the defined terms may occur in combinations, and it is to be understood that the first mentioned radical is a substituent on the subsequently mentioned radical, where the point of substitution, i.e. the point of attachment to another part of the molecule, is on the last mentioned of the radicals.

The term "treatment" is defined as the management and care of a patient for the purpose of combating or alleviating the disease, condition or disorder, and the term includes the administration of the active compound to prevent the onset of the symptoms or complications, or alleviating the symptoms or complications, or eliminating the disease, condition, or disorder.

The term "pharmaceutically acceptable" is defined as being suitable for administration to humans without adverse events.

### SUMMARY OF THE INVENTION

In one aspect of the invention substituted benzamide based inhibitors that modulate the activity of 11βHSD1 leading to altered intracellular concentrations of active glucocorticoid, are provided. More specifically, the present compounds inhibit the activity of 11βHSD1 leading to decreased intracellular concentrations of active glucocorticoid. Thus, the present compounds can be used to treat disorders where a decreased level of active intracellular glucocorticoid is desirable, such as e.g. the metabolic syndrome, type 2 diabetes, impaired glucose tolerance (IGT), impaired fasting glucose (IFG), dyslipidemia, obesity, hypertension, diabetic late complications, cardiovascular diseases, arteriosclerosis, atherosclerosis, myopathy, muscle wasting, osteoporosis, neurodegenerative and psychiatric disorders, and adverse effects of treatment or therapy with glucocorticoid receptor agonists.

One object of the present invention is to provide compounds, pharmaceutical compositions and use of compounds that modulate the activity of 11βHSD1.

The present invention furthermore relates to the use in therapy of the compounds according to the invention, to pharmaceutical compositions comprising the compounds, to the use of said compounds in the manufacture of medicaments, and to the use in therapeutic methods comprising the administration of said compounds

### DETAILED DESCRIPTION OF THE INVENTION

The invention provides for a compound of the general formula (I): 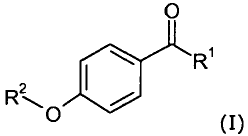 wherein R¹ is selected from the group consisting of: 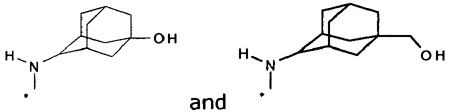
wherein the symbol * denotes the point of attachment,
R² is selected from the group consisting of phenyl substituted with one or two independently selected R³ and pyridinyl substituted with one or two independently selected R³;
R³ is selected from the group consisting of halogen, cyano, -C(=O)OH, -C(=O)R⁴, -CH(OH)R⁴, C(=O)-NR⁶R⁷, -OR⁴, -SR⁴, -S(=O)₂R⁴, -S(=O)₂-NR⁶R⁷, -C=CR⁴R⁵, -C≡C-R⁴, -C₃-C₁₀heterocyclyl optionally substituted with halogen or methyl, a saturated carbocyclic ring having from 3 to 10 carbon atoms optionally containing one or two carbon bridges wherein the carbocyclic ring is optionally substituted with halogen, methyl or hydroxy, phenyl optionally substituted with -C(=O)OH, halogen or methyl, C₁-C₆alkyl optionally substituted with R⁴ and heteroaryl optionally substituted with -C(=O)OH, halogen or methyl;
R⁴ is selected from the group consisting of hydrogen, C₁-C₆alkyl, phenyl, heteroaryl and a saturated carbocyclic ring having from 3 to 10 carbon atoms optionally containing one or two carbon bridges, wherein said C₁-C₆alkyl, phenyl, heteroaryl and saturated carbocyclic ring are optionally substituted with -C(=O)OH, -CH₂OH, halogen, methyl, triflouromethyl, methoxy or hydroxyl and -C(=O)NH₂;
R⁵ is selected from the group consisting of hydrogen, C₁-C₆alkyl, phenyl, heteroaryl anda saturated carbocyclic ring having from 3 to 10 carbon atoms optionally containing one or two carbon bridges, wherein said C₁-C₆alkyl, phenyl, heteroaryl and carbocyclic ring are optionally substituted with -C(=O)OH, -CH₂OH, halogen, methyl, triflouromethyl, methoxy or hydroxy;
R⁶ is selected from the group consisting of hydrogen, C₁-C₆alkyl, tetrahydropyranyl and a saturated carbocyclic ring having from 3 to 10 carbon atoms optionally containing one or two carbon bridges, wherein said C₁-C₆alkyl, tetrahydropyranyl, and carbocyclic ring are optionally substituted with one or two substituents independently selected from the group consisting of halogen and hydroxy;
R⁷ is selected from the group consisting of hydrogen, C₁-C₆alkyl, tetrahydropyranyl and a saturated carbocyclic ring having from 3 to 10 carbon atoms optionally containing one or two carbon bridges, wherein said C₁-C₆alkyl, tetrahydropyranyl, and carbocyclic ring are optionally substituted with one or two substituents independently selected from the group consisting of halogen and hydroxy;
or R⁶ and R⁷ together with the nitrogen atom to which they are attached form a piperidine or a pyrrolidine ring, wherein said ring is optionally substituted with hydroxy or halogen;
or a salt thereof with a pharmaceutically acceptable acid or base, or any optical isomer or mixture of optical isomers, including a racemic mixture, or any tautomeric forms.

In another aspect of the invention, R¹ is

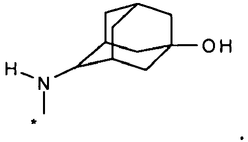

In a further aspect of the invention, R¹ is

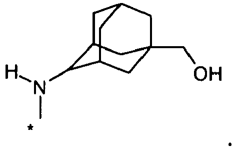

In one aspect of the invention, R² is phenyl substituted with one or two independently selected R³.

In a further aspect of the invention, R² is pyridinyl substituted with one or two independently selected R³.

In one aspect of the invention, R² is substituted with one R³.

In another aspect of the invention, R² is substituted with two independently selected R³.

In one aspect of the invention, R³ is selected from the group consisting of cyano and halogen.

In one aspect of the invention, R² is substituted with one R³, which is cyano.

In one aspect of the invention, R² is substituted with two R³, which are each halogen.

In one aspect of the invention, R³ is chlorine.

In one aspect of the invention, R⁴ is selected from the group consisting of hydrogen, methyl, ethyl, phenyl, pyridinyl, imidazolyl, cyclopropyl, cyclobutyl and cyclohexyl, wherein said methyl, ethyl, phenyl, pyridinyl, imidazolyl, cyclopropyl, cyclobutyl and cyclohexyl are optionally substituted with -C(=O)OH, -CH₂OH, chlorine, fluorine, methyl, triflouromethyl, methoxy or hydroxy.

In one aspect of the invention, R⁵ is selected from the group consisting of hydrogen, methyl, ethyl, phenyl, pyridinyl, imidazolyl, cyclopropyl, cyclobutyl and cyclohexyl, wherein said methyl, ethyl, phenyl, pyridinyl, imidazolyl, cyclopropyl, cyclobutyl and cyclohexyl are optionally substituted with -C(=O)OH, -CH₂OH, chlorine, fluorine, methyl, triflouromethyl, methoxy or hydroxy.

In another aspect of the invention, R⁶ is selected from the group consisting of hydrogen, methyl, ethyl, tetrahydropyranyl, cyclopropyl, cyclobutyl and cyclohexyl, wherein said methyl, ethyl, tetrahydropyranyl, cyclopropyl, cyclobutyl, and cyclohexyl are optionally substituted with one or two substituents independently selected from the group consisting of chlorine, fluorine and hydroxy.

In another aspect of the invention, R⁷ is selected from the group consisting of hydrogen, methyl, ethyl, tetrahydropyranyl, cyclopropyl, cyclobutyl and cyclohexyl, wherein said methyl, ethyl, tetrahydropyranyl, cyclopropyl, cyclobutyl, and cyclohexyl are optionally substituted with one or two substituents independently selected from the group consisting of chlorine, fluorine and hydroxy.

In another aspect of the invention, R⁶ and R⁷ together with the nitrogen atom to which they are attached form a piperidine or a pyrrolidine ring, wherein said ring is optionally substituted with hydroxy, chlorine or fluorine.

In another aspect of the invention, heteroaryl is selected from the group consisting of isoxazolyl, pyridazinyl, pyrimidinyl, imidazolyl, pyrazolyl and pyridinyl.

In another aspect of the invention, the saturated carbocyclic ring having from 3 to 10 carbon atoms is selected from the group consisting of cyclopropyl, cyclobutyl, cyclopentyl and cyclohexyl.

In another aspect of the invention, C₃-C₁₀heterocyclyl is selected from the group consisting of pyrrolidinyl, tetrahydropyranyl, piperidinyl, piperazinyl, and morpholinyl.

In another aspect of the invention, a compound is selected from the group consisting of 4-(2,4-Dichloro-phenoxy)-N-(5-hydroxy-adamantan-2-yl)-benzamide, 4-(5-Cyano-pyridin-2-yloxy)-N-(5-hydroxy-adamantan-2-yl)-benzamide, 4-(5-Chloro-pyridin-2-yloxy)-N-(5-hydroxy-adamantan-2-yl)-benzamide, 4-(5-Chloro-pyridin-2-yloxy)-N-(5-hydroxymethyl-adamantan-2-yl)-benzamide, and 4-(5-Chloro-pyridin-2-yloxy)-N-(4-hydroxymethyl-cyclohexyl)-N-methyl-benzamide.

In another aspect of the invention the compound is 4-(5-Chloro-pyridin-2-yloxy)-N-(5-hydroxy-adamantan-2-yl)-benzamide or a pharmaceutically acceptable salt thereof.

In another aspect of the invention the compound is 4-(5-Chloro-pyridin-2-yloxy)-N-(5-hydroxymethyl-adamantan-2-yl)-benzamide or a pharmaceutically acceptable salt thereof.

In one aspect of the invention, R² is pyridine-2-yl substituted with one or two independently selected R³. In a further aspect, pyridine-2-yl is substituted with one R³ selected from the group consisting of chlorine and cyano, such as 5-chloro or 5-cyano.

In one embodiment the compound of the invention is an agent useful for the treatment, prevention and/or prophylaxis of any conditions, disorders and diseases wherein a modulation or an inhibition of the activity of 11βHSD1 is beneficial.

In one embodiment the compound of the invention is an agent useful for the treatment, prevention and/or prophylaxis of any conditions, disorders and diseases that are influenced by intracellular glucocorticoid levels.

In one embodiment the compound of the invention is an agent useful for the treatment, prevention and/or prophylaxis of conditions, disorders or diseases selected from the group consisting of the metabolic syndrome, insulin resistance, dyslipidemia, hypertension and obesity.

In one embodiment the compound of the invention is an agent useful for the treatment, prevention and/or prophylaxis of type 2 diabetes, impaired glucose tolerance (IGT), impaired fasting glucose (IFG).

In one embodiment the compound of the invention is an agent useful for the delaying or prevention of the progression from IGT into type 2 diabetes.

In one embodiment the compound of the invention is an agent useful for delaying or prevention of the progression of the metabolic syndrome into type 2 diabetes.

In one embodiment the compound of the invention is an agent useful for the treatment, prevention and/or prophylaxis of adverse effects of glucocorticoid receptor agonist treatment or therapy.

In one aspect, the invention relates to a pharmaceutical composition comprising, as an active ingredient, at least one compound according to the invention together with one ore more pharmaceutically acceptable carriers or excipients.

In one aspect, the invention relates to a pharmaceutical composition which is for oral, nasal, buccal, transdermal, pulmonal or parenteral administration.

In one aspect, the invention relates to a pharmaceutical composition in unit dosage form, comprising from 0.05 mg to 2000 mg/day, from 0.1 mg to 1000 mg or from 0.5 mg to 500 mg per day of the compound according to the invention.

In one aspect, the invention relates to a use of a compound according to the invention for the preparation of a pharmaceutical composition for the treatment, prevention and/or prophylaxis of any conditions, disorders and diseases wherein a modulation or an inhibition of the activity of 11βHSD1 is beneficial. In another aspect, the invention relates to a use of a compound according to the invention for the preparation of a pharmaceutical composition for the treatment, prevention and/or prophylaxis of any conditions, disorders and diseases that are influenced by intracellular glucocorticoid levels.

In one aspect, the invention relates to a compound according to the invention for treatment, prevention and/or prophylaxis of any conditions, disorders and diseases wherein a modulation or an inhibition of the activity of 11βHSD1 is beneficial.

In one aspect, the invention relates to a compound according to the invention for treatment, prevention and/or prophylaxis of any conditions, disorders and diseases that are influenced by intracellular glucocorticoid levels.

In one aspect, the invention relates to a compound according to the invention for treatment, prevention and/or prophylaxis of conditions, disorders or diseases selected from the group consisting of the metabolic syndrome, insulin resistance, dyslipidemia, hypertension and obesity.

In one aspect, the invention relates to a compound according to the invention for treatment, prevention and/or prophylaxis of type 2 diabetes, impaired glucose tolerance (IGT), impaired fasting glucose (IFG).

In one aspect, the invention relates to a compound according to the invention for delaying or prevention of the progression from IGT to type 2 diabetes.

In one aspect, the invention relates to a compound according to the invention for delaying or prevention of the progression of the metabolic syndrome into type 2 diabetes.

In one aspect, the invention relates to a compound according to the invention for treatment, prevention and/or prophylaxis of adverse effects of glucocorticoid receptor agonist treatment or therapy.

In one aspect the invention relates to the use of the compounds in the treatment, prevention and/or prophylaxis of any conditions, disorders or diseases wherein a modulation or an inhibition of the activity of 11βHSD1 is beneficial, the method comprising administering to a subject in need thereof an effective amount of a compound according to the invention. In a further aspect, the conditions, disorders or diseases are selected from the group consisting of the metabolic syndrome, insulin resistance, dyslipidemia, hypertension and obesity.

In one aspect of the invention, the compounds according to the invention have a IC₅₀ value as tested as described under the heading "PHARMACOLOGICAL METHODS" below 1500nM, in a further aspect below 500nM, in yet a further aspect below 300 nM and in yet a further aspect below 200 nM.

Some of the compounds of the present invention have asymmetric centers and may occur as racemates, racemic mixtures, and as individual enantiomers or diastereoisomers, with all isomeric forms being included in the present invention as well as mixtures thereof.

The present invention also encompasses pharmaceutically acceptable salts of the present compounds. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxyl-naphthoates, glycerophosphates, ketoglutarates and the like. Further examples of pharmaceutically acceptable inorganic or organic acid addition salts include the pharmaceutically acceptable salts listed in J. Pharm. Sci., 66, 2 (1977). Examples of metal salts include lithium, sodium, potassium, barium, calcium, magnesium, zinc, calcium salts and the like. Examples of amines and organic amines include ammonium, methylamine, dimethylamine, trimethylamine, ethylamine, diethylamine, propylamine, butylamine, tetramethylamine, ethanolamine, diethanolamine, triethanolamine, meglumine, ethylenediamine, choline, N,N'-dibenzylethylenediamine, N-benzylphenylethylamine, N-methyl-D-glucamine, guanidine and the like. Examples of cationic amino acids include lysine, arginine, histidine and the like.

Further, some of the compounds of the present invention may form solvates with water or common organic solvents. Such solvates are encompassed within the scope of the invention.

The pharmaceutically acceptable salts are prepared by reacting a compound of the present invention with 1 to 4 equivalents of a base such as sodium hydroxide, sodium methoxide, sodium hydride, potassium tert-butoxide, calcium hydroxide, magnesium hydroxide and the like, in solvents like ether, THF, methanol, tert-butanol, dioxane, isopropanol, ethanol etc. Mixtures of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, enzymatic resolution, resolving the diastereomeric salts formed with chiral acids such as mandelic acid, camphorsulfonic acid, tartaric acid, lactic acid, and the like wherever applicable or chiral bases such as brucine, (R)- or (S)-phenylethylamine, cinchona alkaloids and their derivatives and the like. Commonly used methods are compiled by Jaques et al. in "Enantiomers, Racemates and Resolution" (Wiley Interscience, 1981). More specifically the compound of the present invention may be converted to a 1:1 mixture of diastereomeric amides by treating with chiral amines, aminoacids, aminoalcohols derived from aminoacids; conventional reaction conditions may be employed to convert acid into an amide; the diastereomers may be separated either by fractional crystallization or chromatography and the stereoisomers of compound of formula I may be prepared by hydrolysing the pure diastereomeric amide.

Various polymorphs of the compounds forming part of this invention may be prepared by crystallization of said compounds under different conditions. For example, using different solvents commonly used or their mixtures for recrystallization; crystallizations at different temperatures; various modes of cooling, ranging from very fast to very slow cooling during crystallizations. Polymorphs may also be obtained by heating or melting the compound followed by gradual or fast cooling. The presence of polymorphs may be determined by solid probe nmr spectroscopy, ir spectroscopy, differential scanning calorimetry, powder X-ray diffraction or such other techniques.

It is a well known problem in drug discovery that compounds, such as enzyme inhibitors, may be very potent and selective in biochemical assays, yet be inactive *in vivo.* This lack of so-called bioavailability may be ascribed to a number of different factors such as lack of or poor absorption in the gut, first pass metabolism in the liver and/or poor uptake in cells. Although the factors determining bioavailability are not completely understood, there are many examples in the scientific literature - well known to those skilled in the art - of how to modify compounds, which are potent and selective in biochemical assays but show low or no activity *in vivo,* into drugs that are biologically active.

Examples of said modifications, which are not intended in any way to limit the scope of the invention, include changing of one or more carboxy groups to esters (for instance methyl esters, ethyl esters, tert-butyl, acetoxymethyl, pivaloyloxymethyl esters or other acyloxymethyl esters). Compounds of the invention, original compounds, such modified by attaching chemical groups are termed 'modified compounds'.

The compounds according to the invention alter, and more specifically, reduce the level of active intracellular glucocorticoid and are accordingly useful for the treatment, prevention and/or prophylaxis of disorders and diseases in which such a modulation or reduction is beneficial.

Accordingly, the present compounds may be applicable for the treatment, prevention and/or prophylaxis of the metabolic syndrome, insulin resistance, dyslipidemia, hypertension, obesity, type 2 diabetes, impaired glucose tolerance (IGT), impaired fasting glucose (IFG), Latent Autoimmune Diabetes in the Adult (LADA), type 1 diabetes, diabetic late complications including cardiovascular diseases, cardiovascular disorders, disorders of lipid metabolism, neurodegenerative and psychiatric disorders, dysregulation of intraocular pressure including glaucoma, immune disorders, inappropriate immune responses, musculo-skeletal disorders, gastrointestinal disorders, polycystic ovarie syndrome (PCOS), reduced hair growth or other diseases, disorders or conditions that are influenced by intracellular glucocorticoid levels, adverse effects of increased blood levels of active endogenous or exogenous glucocorticoid, and any combination thereof, adverse effects of increased plasma levels of endogenous active glucocorticoid, Cushing's disease, Cushing's syndrome, adverse effects of glucocorticoid receptor agonist treatment of autoimmune diseases, adverse effects of glucocorticoid receptor agonist treatment of inflammatory diseases, adverse effects of glucocorticoid receptor agonist treatment of diseases with an inflammatory component, adverse effects of glucocorticoid receptor agonist treatment as a part of cancer chemotherapy, adverse effects of glucocorticoid receptor agonist treatment for surgical/post-surgical or other trauma, adverse effects of glucocorticoid receptor agonist therapy in the context of organ or tissue transplantation or adverse effects of glucocorticoid receptor agonist treatment in other diseases, disorders or conditions where glucocorticoid receptor agonists provide clinically beneficial effects.

More specifically the present compounds may be applicable for the treatment, prevention and/or prophylaxis of the metabolic syndrome, type 2 diabetes, diabetes as a consequence of obesity, insulin resistance, hyperglycemia, prandial hyperglycemia, hyperinsulinemia, inappropriately low insulin secretion, impaired glucose tolerance (IGT), impaired fasting glucose (IFG), increased hepatic glucose production, type 1 diabetes, LADA, pediatric diabetes, dyslipidemia, diabetic dyslipidemia, hyperlipidemia, hypertriglyceridemia, hyperlipoproteinemia, hypercholesterolemia, decreased HDL cholesterol, impaired LDL/HDL ratio, other disorders of lipid metabolism, obesity, visceral obesity, obesity as a consequence of diabetes, increased food intake, hypertension, diabetic late complications, micro-/macro-albuminuria, nephropathy, retinopathy, neuropathy, diabetic ulcers, cardiovascular diseases, arteriosclerosis, atherosclerosis, coronary artery disease, cardiac hypertrophy, myocardial ischemia, heart insufficiency, congestional heart failure, stroke, myocardial infarction, arrythmia, decreased blood flow, erectile dysfunction (male or female), myopathy, loss of muscle tissue, muscle wasting, muscle catabolism, osteoporosis, decreased linear growth, neurodegenerative and psychiatric disorders, Alzheimers disease, neuronal death, impaired cognitive function, depression, anxiety, eating disorders, appetite regulation, migraine, epilepsia, addiction to chemical substances, disorders of intraocular pressure, glaucoma, polycystic ovary syndrome (PCOS), inappropriate immune responses, inappropriate T helper-1/T helper-2 polarisation, bacterial infections, mycobacterial infections, fungal infections, viral infections, parasitic infestations, suboptimal responses to immunizations, immune dysfunction, partial or complete baldness, or other diseases, disorders or conditions that are influenced by intracellular glucocorticoid levels and any combination thereof, adverse effects of glucocorticoid receptor agonist treatment of allergic-inflammatory diseases such as asthma and atopic dermatitis, adverse effects of glucocorticoid receptor agonist treatment of disorders of the respiratory system e.g. asthma, cystic fibrosis, emphysema, bronchitis, hypersensitivity, pneumonitis, eosinophilic pneumonias, pulmomary fibrosis, adverse effects of glucocorticoid receptor agonist treatment of inflammatory bowel disease such as Crohn's disease and ulcerative colitis; adverse effects of glucocorticoid receptor agonist treatment of disorders of the immune system, connective tissue and joints e.g. reactive arthritis, rheumatoid arthritis, Sjögren's syndrome, systemic lupus erythematosus, lupus nephritis, Henoch-Schönlein purpura, Wegener's granulomatosis, temporal arteritis, systemic sclerosis, vasculitis, sarcoidosis, dermatomyositis-polymyositis, pemphigus vulgaris; adverse effects of glucocorticoid receptor agonist treatment of endocrinological diseases such as hyper-thyroidism, hypoaldosteronism, hypopituitarism; adverse effects of glucocorticoid receptor agonist treatment of hematological diseases e.g. hemolytic anemia, thrombocytopenia, paroxysmal nocturnal hemoglobinuria; adverse effects of glucocorticoid receptor agonist treatment of cancer such as spinal cord diseases, neoplastic compression of the spinal cord, brain tumours, acute lymphoblastic leukemia, Hodgkin's disease, chemotherapy-induced nausea, adverse effects of glucocorticoid receptor agonist treatment of diseases of muscle and at the neuro-muscular joint e.g. myasthenia gravis and heriditary myopathies (e.g. Duchenne muscular dystrophy), adverse effects of glucocorticoid receptor agonist treatment in the context of surgery & transplantation e.g. trauma, post-surgical stress, surgical stress, renal transplantation, liver transplantation, lung transplanttation, pancreatic islet transplantation, blood stem cell transplantation, bone marrow transplantation, heart transplantation, adrenal gland transplantation, tracheal transplanttation, intestinal transplantation, corneal transplantation, skin grafting, keratoplasty, lens implanttation and other procedures where immunosuppression with glucocorticoid receptor agonists is beneficial; adverse effects of glucocorticoid receptor agonist treatment of brain absess, nausea/vomiting, infections, hypercalcemia, adrenal hyperplasia, autoimmune hepatitis, spinal cord diseases, saccular aneurysms or adverse effects to glucocorticoid receptor agonist treatment in other diseases, disorders and conditions where glucocorticoid receptor agonists provide clinically beneficial effects.

Accordingly, in a further aspect the invention relates to a compound according to the invention for use as a pharmaceutical composition.

The invention also relates to pharmaceutical compositions comprising, as an active ingredient, at least one compound according to the invention together with one or more pharmaceutically acceptable carriers or diluents.

The pharmaceutical composition is preferably in unit dosage form, comprising from about 0.05 mg/day to about 2000 mg/day, preferably from about 1mg/day to about 500 mg/day of a compound according to the invention.

In another embodiment, the patient is treated with a compound according to the invention for at least about 1 week, for at least about 2 weeks, for at least about 4 weeks, for at least about 2 months or for at least about 4 months.

In yet another embodiment, the pharmaceutical composition is for oral, nasal, transdermal, pulmonal or parenteral administration.

Furthermore, the invention relates to the use of a compound according to the invention for the preparation of a pharmaceutical composition for the treatment, prevention and/or prophylaxis of disorders and diseases wherein a modulation or an inhibition of the activity of 11βHSD1 is beneficial.

The invention also relates to the use of the compounds in a method for the treatment, prevention and/or prophylaxis of disorders and diseases wherein a modulation or an inhibition of the activity of 11βHSD1 is beneficial, the method comprising administering to a subject in need thereof an effective amount of a compound according to the invention.

In a preferred embodiment of the invention the present compounds are used for the preparation of a medicament for the treatment, prevention and/or prophylaxis of any diseases and conditions that are influenced by intracellular glucocorticoid levels as mentioned above.

Thus, in a preferred embodiment of the invention the present compounds are used for the preparation of a medicament for the treatment, prevention and/or prophylaxis of conditions and disorders where a decreased level of active intracellular glucocorticoid is desirable, such as the conditions and diseases mentioned above.

In yet a preferred embodiment of the invention the present compounds are used for the preparation of a medicament for the treatment, prevention and/or prophylaxis of the metabolic syndrome including insulin resistance, dyslipidemia, hypertension and obesity.

In yet another preferred embodiment of the invention the present compounds are used for the preparation of a medicament for the treatment, prevention and/or prophylaxis of type 2 diabetes, impaired glucose tolerance (IGT), impaired fasting glucose (IFG).

In yet another preferred embodiment of the invention the present compounds are used for the preparation of a pharmaceutical composition for the delaying or prevention of the progression from IGT to type 2 diabetes.

In yet another preferred embodiment of the invention the present compounds are used for the preparation of a pharmaceutical composition for the delaying or prevention of the progression of the metabolic syndrome into type 2 diabetes.

In still another preferred embodiment of the invention the present compounds are used for the preparation of a pharmaceutical composition for the treatment, prevention and/or prophylaxis of diabetic late complications including cardiovascular diseases; arteriosclerosis; atherosclerosis.

In a further preferred embodiment of the invention the present compounds are used for the preparation of a pharmaceutical composition for the treatment, prevention and/or prophylaxis of neurodegenerative and psychiatric disorders.

In yet a further preferred embodiment of the invention the present compounds are used for the preparation of a pharmaceutical composition for the treatment, prevention and/or prophylaxis of adverse effects of glucocorticoid receptor agonist treatment or therapy.

In another embodiment of the present invention, the route of administration may be any route which effectively transports a compound according to the invention to the appropriate or desired site of action, such as oral, nasal, buccal, transdermal, pulmonal, or parenteral.

In still a further aspect of the invention the present compounds are administered in combination with one or more further active substances in any suitable ratios. Such further active substances may e.g. be selected from antiobesity agents, antidiabetics, agents modifying the lipid metabolism, antihypertensive agents, glucocorticoid receptor agonists, agents for the treatment and/or prevention of complications resulting from or associated with diabetes and agents for the treatment and/or prevention of complications and disorders resulting from or associated with obesity.

Thus, in a further aspect of the invention the present compounds may be administered in combination with one or more antiobesity agents or appetite regulating agents.

Such agents may be selected from the group consisting of CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, PPAR (peroxisome proliferator-activated receptor) modulators, RXR (retinoid X receptor) modulators, TR β agonists, AGRP (Agouti related protein) inhibitors, H3 histamine antagonists, opioid antagonists (such as naltrexone), exendin-4, GLP-1 and ciliary neurotrophic factor.

In one embodiment of the invention the antiobesity agent is leptin; dexamphetamine or amphetamine; fenfluramine or dexfenfluramine; sibutramine; orlistat; mazindol or phentermine.

Suitable antidiabetic agents include insulin, insulin analogues and derivatives such as those disclosed in EP 792 290 (Novo Nordisk A/S), e.g. N^{εB29}-tetradecanoyl des (B30) human insulin, EP 214 826 and EP 705 275 (Novo Nordisk A/S), e.g. Asp^{B28} human insulin, US 5,504,188 (Eli Lilly), e.g. Lys^{B28} Pro^{B29} human insulin, EP 368 187 (Aventis), eg Lantus, GLP-1 (glucagon like peptide-1) and GLP-1 derivatives such as those disclosed in WO 98/08871 to Novo Nordisk A/S, as well as orally active hypoglycaemic agents.

The orally active hypoglycaemic agents preferably comprise sulphonylureas, biguanides, meglitinides, glucosidase inhibitors, glucagon antagonists such as those disclosed in WO 99/01423 to Novo Nordisk A/S and Agouron Pharmaceuticals, Inc., GLP-1 agonists, potassium channel openers such as those disclosed in WO 97/26265 and WO 99/03861 to Novo Nordisk A/S, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as PPARα modulators, PPARδ modulators, cholesterol absorption inhibitors, HSL (hormone-sensitive lipase) inhibitors and HMG CoA inhibitors (statins), nicotinic acid, fibrates, anion exchangers, compounds lowering food intake, bile acid resins, RXR agonists and agents acting on the ATP-dependent potassium channel of the β-cells.

In one embodiment, the present compounds are administered in combination with insulin or an insulin analogue or derivative, such as N^{εB29}-tetradecanoyl des (B30) human insulin, Asp^{B26} human insulin, Lys^{B28} Pro^{B29} human insulin, Lantus®, or a mix-preparation comprising one or more of these.

In a further embodiment the present compounds are administered in combination with a sulphonylurea e.g. tolbutamide, glibenclamide, glipizide or glicazide.

In another embodiment the present compounds are administered in combination with a biguanide e.g. metformin.

In yet another embodiment the present compounds are administered in combination with a meglitinide e.g. repaglinide or senaglinide.

In still another embodiment the present compounds are administered in combination with a thiazolidinedione e.g. troglitazone, ciglitazone, pioglitazone, rosiglitazone or compounds disclosed in WO 97/41097 such as 5-[[4-[3-Methyl-4-oxo-3,4-dihydro-2-quinazolinyl]-methoxy]phenyl-methyl]thiazolidine-2,4-dione or a pharmaceutically acceptable salt thereof, preferably the potassium salt.

In yet another embodiment the present compounds may be administered in combination with the insulin sensitizers disclosed in WO 99/19313 such as (-) 3-[4-[2-Phenoxazin-10-yl)ethoxy]phenyl]-2-ethoxypropanoic acid or a pharmaceutically acceptable salts thereof, preferably the arginine salt.

In a further embodiment the present compounds are administered in combination with an α-glucosidase inhibitor e.g. miglitol or acarbose.

In another embodiment the present compounds are administered in combination with an agent acting on the ATP-dependent potassium channel of the β-cells e.g. tolbutamide, glibenclamide, glipizide, glicazide or repaglinide.

Furthermore, the present compounds may be administered in combination with nateglinide.

In still another embodiment the present compounds are administered in combination with an antihyperlipidemic agent or antilipidemic agent e.g. cholestyramine, colestipol, clofibrate, gemfibrozil, fenofibrate, bezafibrate, tesaglitazar, EML-4156, LY-818, MK-767, atorvastatin, fluvastatin, lovastatin, pravastatin, simvastatin, acipimox, probucol, ezetimibe or dextrothyroxine.

In a further embodiment the present compounds are administered in combination with more than one of the above-mentioned compounds e.g. in combination with a sulphonylurea and metformin, a sulphonylurea and acarbose, repaglinide and metformin, insulin and a sulphonylurea, insulin and metformin, insulin, insulin and lovastatin, etc.

Further, the present compounds may be administered in combination with one or more antihypertensive agents. Examples of antihypertensive agents are β-blockers such as alprenolol, atenolol, timolol, pindolol, propranolol, metoprolol, bisoprololfumerate, esmolol, acebutelol, metoprolol, acebutolol, betaxolol, celiprolol, nebivolol, tertatolol, oxprenolol, amusolalul, carvedilol, labetalol, β2-receptor blockers e.g. S-atenolol, OPC-1085, ACE (angiotensin converting enzyme) inhibitors such as quinapril, lisinopril, enalapril, captopril, benazepril, perindopril, trandolapril, fosinopril, ramipril, cilazapril, delapril, imidapril, moexipril, spirapril, temocapril, zofenopril, S-5590, fasidotril, Hoechst-Marion Roussel: 100240 (EP 00481522), omapatrilat, gemopatrilat and GW-660511, calcium channel blockers such as nifedipine, felodipine, nicardipine, isradipine, nimodipine, diltiazem, amlodipine, nitrendipine, verapamil, lacidipine, lercanidipine, aranidipine, cilnidipine, clevidipine, azelnidipine, barnidipine, efonodipine, iasidipine, iemildipine, iercanidipine, manidipine, nilvadipine, pranidipine, furnidipine, α-blockers such as doxazosin, urapidil, prazosin, terazosin, bunazosin and OPC-28326, diuretics such as thiazides/sulphonamides (e.g. bendroflumetazide, chlorothalidone, hydrochlorothiazide and clopamide), loop-diuretics (e.g. bumetanide, furosemide and torasemide) and potassium sparing diuretics (e.g. amiloride, spironolactone), endothelin ET-A antagonists such as ABT-546, ambrisetan, atrasentan, SB-234551, CI-1034, S-0139 and YM-598, endothelin antagonists e.g. bosentan and J-104133, renin inhibitors such as aliskiren, vasopressin V1 antagonists e.g. OPC-21268, vasopressin V2 antagonists such as tolvaptan, SR-121463 and OPC-31260, B-type natriuretic peptide agonists e.g. Nesiritide, angiotensin II antagonists such as irbesartan, candesartancilexetil, losartan, valsartan, telmisartan, eprosartan, candesartan, CL-329167, eprosartan, iosartan, olmesartan, pratosartan, TA-606, and YM-358, 5-HT2 agonists e.g. fenoldopam and ketanserin, adenosine A1 antagonists such as naftopidil, N-0861 and FK-352, thromboxane A2 antagonists such as KT2-962, endopeptidase inhibitors e.g. ecadotril, nitric oxide agonists such as LP-805, dopamine D1 antagonists e.g. MYD-37, dopamine D2 agonists such as nolomirole, n-3 fatty acids e.g. omacor, prostacyclin agonists such as treprostinil, beraprost, PGE1 agonists e.g. ecraprost, Na+/K+ ATPase modulators e.g. PST-2238, Potassium channel activators e.g. KR-30450, vaccines such as PMD-3117, Indapamides, CGRP-unigene, guanylate cyclase stimulators, hydralazines, methyldopa, docarpamine, moxonidine, CoAprovel, MondoBiotech-811.

Further reference can be made to Remington: The Science and Practice of Pharmacy, 19th Edition, Gennaro, Ed., Mack Publishing Co., Easton, PA, 1995.

Furthermore, the present compounds may be administered in combination with one or more glucocorticoid receptor agonists. Examples of such glucocorticoid receptor agonists are betametasone, dexamethasone, hydrocortisone, methylprednisolone, prednisolone, prednisone, beclomethasone, butixicort, clobetasol, flunisolide, flucatisone (and analogues), momethasone, triamcinolonacetonide, triamcinolonhexacetonide GW-685698, NXC-1015, NXC-1020, NXC-1021, NS-126, P-4112, P-4114, RU-24858 and T-25 series.

It should be understood that any suitable combination of the compounds according to the invention with one or more of the above-mentioned compounds and optionally one or more further pharmacologically active substances are considered to be within the scope of the present invention.

### PHARMACEUTICAL COMPOSITIONS

The compounds of the present invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy,19th Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well-known in the art.

Liquid dosage forms for oral administration include solutions, emulsions, suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention. Other suitable administration forms include suppositories, sprays, ointments, crèmes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 2000 mg, e.g. from about 0.1 to about 1000 mg, from about 0.5 mg to about 500 mg., from about 1 mg to about 200 mg, e.g. about 100 mg.

For parenteral routes, such as intravenous, intrathecal, intramuscular and similar administration, typically doses are in the order of about half the dose employed for oral administration.

The compounds of this invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of the compounds for use according to the present invention which are generally prepared by reacting the free base with a suitable organic or inorganic acid or by reacting the acid with a suitable organic or inorganic base. When a compound for use according to the present invention, contains a free base such salts are prepared in a conventional manner by treating a solution or suspension of the compound with a chemical equivalent of a pharmaceutically acceptable acid. When a compounds for use according to the present invention, contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds for use according to the present invention and these form a further aspect of the present invention.

For parenteral administration, solutions of the present compounds in sterile aqueous solution, aqueous propylene glycol or sesame or peanut oil may be employed. Such aqueous solutions should be suitable buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of suitable carriers are water, salt solutions, alcohols, polyethylene glycols, polyhydroxyethoxylated castor oil, peanut oil, olive oil, syrup, phosphor-lipids, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents.

The pharmaceutical compositions formed by combining the compounds of the invention and the pharmaceutically acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. These formulations may be in the form of powder or granules, as a solution or suspension in an aqueous or non-aqueous liquid, or as an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or a soft gelatine capsule wherein the active ingredient is mixed with water or an oil medium, for example peanut oil, liquid paraffin, or olive oil.

Aqueous suspensions may contain the active compounds in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions comprising a compound for use according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, preservative and flavouring and colouring agent. The pharmaceutical compositions may be in the form of a sterile injectable aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the present invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the present invention are contemplated. For the purpose of this application, topical applications shall include mouth washes and gargles.

The compounds for use according to the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds for use according to the present invention may form solvates with water or common organic solvents. Such solvates are also encompassed within the scope of the present invention.

Thus, in a further embodiment, there is provided a pharmaceutical composition comprising a compound for use according to the present invention, or a pharmaceutically acceptable salt, solvate, and one or more pharmaceutically acceptable carriers, excipients, or diluents.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

A typical tablet which may be prepared by conventional tabletting techniques may contain:

| Core: | |
|---|---|
| Active compound (as free compound or salt thereof) | 5.0 mg |
| Lactosum Ph. Eur. | 67.8 mg |
| Cellulose, microcryst. (Avicel) | 31.4 mg |
| Amberlite^{®□}IRP88* | 1.0 mg |
| Magnesii stearas Ph. Eur. | q.s. |
| | |

| Coating: | |
|---|---|
| Hydroxypropyl methylcellulose | approx. 9 mg |
| Mywacett 9-40 T** | approx. 0.9 mg |

| | |
|---|---|
| * Polacrillin potassium NF, tablet disintegrant, Rohm and Haas. ** Acylated monoglyceride used as plasticizer for film coating. | |

The compounds of the invention may be administered to a patient which is a mammal, especially a human in need thereof. Such mammals include also animals, both domestic animals, e.g. household pets, and non-domestic animals such as wildlife.

The present invention also relates to the below methods of preparing the compounds of the invention.

The features disclosed in the foregoing description may, both separately and in any combination thereof, be material for realising the invention in diverse forms thereof.

All headings and sub-headings are used herein for convenience only and should not be construed as limiting the invention in any way.

Any combination of the above-described elements in all possible variations thereof is encompassed by the invention unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

All methods described herein can be performed in any suitable order unless otherwise indicated herein or otherwise clearly contradicted by context.

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents,

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a formulation described herein as comprising a particular element should be understood as also describing a formulation consisting of that element, unless otherwise stated or clearly contradicted by context).

This invention includes all modifications and equivalents of the subject matter recited in the aspects or claims presented herein to the maximum extent permitted by applicable law.

The present invention is further illustrated in the following representative examples which are, however, not intended to limit the scope of the invention in any way.

### EXAMPLES

The following examples and general procedures refer to intermediate compounds and final products of general formula (I) identified in the specification and in the synthesis schemes. The preparation of the compounds of general formula (I) of the present invention is described in detail using the following examples. Occasionally, the reaction may not be applicable as described to each compound included within the disclosed scope of the invention. The compounds for which this occurs will be readily recognised by those skilled in the art. In these cases, the reactions can be successfully performed by conventional modifications known to those skilled in the art, which is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) downfield from tetramethylsilane as internal reference standard. M.p. is melting point and is given in °C and uncorrected. Column chromatography was carried out using the technique described by W.C. Still et al., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.

**Preparative HPLC: Column:** 1.9 x 15 cm Waters XTerra RP-18. Buffer: linear gradient 5 - 95 % MeCN in water over 15 min, 0.1 % TFA, flow rate of 15 ml/min. The pooled fractions are either evaporated to dryness *in vacuo,* or evaporated *in vacuo* until the MeCN is removed, and then frozen and freeze-dried.

### LC-MS.

The following instrumentation was used:
● Hewlett Packard series 1100 G1312A Bin Pump
● Hewlett Packard series 1100 Column compartment
● Hewlett Packard series 1100 G13 15A DAD diode array detector
● Hewlett Packard series 1100 MSD

The instrument was controlled by HP Chemstation software.

The HPLC pump was connected to two eluent reservoirs containing:
- A:: 0.01% TFA in water
- B:: 0.01% TFA in acetonitrile

The analysis was performed at 40 °C by injecting an appropriate volume of the sample (preferably 1 µl) onto the column, which is eluted with a gradient of acetonitrile.

The HPLC conditions, detector settings and mass spectrometer settings which were used are as follows:
- Column: Waters Xterra MS C-18 X 3 mm id
- Gradient: 10% - 100% acetonitrile linear during 7.5 min at 1.0ml/min
- Detection: 210 nm (analogue output from DAD)
- MS: Ionisation mode API-ES, Scan 100-1000 amu step 0.1 amu

The abbreviations as used in the examples have the following meaning:
- ADDP:: 1,1'-(Azodicarbonyl)dipiperidine
- CDCl₃:: Deuterio chloroform
- DCM:: Dichloromethane
- DEAD:: 1,1'-Diethyl azodicarboxylate
- DIAD:: 1,1'-Disopropyl azodicarboxylate
- DIC:: N,N'-Diisopropylcarbodiimide
- DMAP:: 4-Dimethylaminopyridine
- DMF:: N, N-Dimethylformamide
- DMSO-d₆:: Hexadeuterio dimethylsulfoxide
- DMSO:: Dimethylsulfoxide
- DIPEA:: Diisopropylethylamine
- EDC:: 1-(3-Dimethylaminopropyl)-3-ethylcarbodiimide hydrochloride
- EtOAc:: Ethyl acetate
- EtOH:: Ethanol
- HOBT:: 1-Hydroxy-benzotriazole
- hrs:: hours
- MCPBA:: meta-Chloroperbenzoic acid
- MeCN :: Acetonitrile
- min:: minutes
- NMP:: N-Methylpyrrolidinone
- TEA:: Triethylamine
- TFA:: Trifluoroacetic acid
- THF:: Tetrahydrofuran
- TLC:: Thin layer chromatography

### General method A:

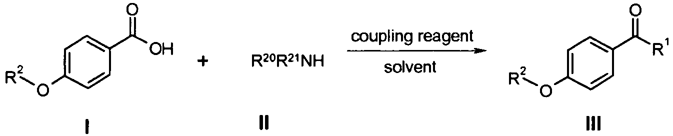

By allowing an acid (I), wherein R² is as defined above, to be coupled with an amine (II), wherein R²⁰ and R²¹ together with the amine that the groups are attached to form R¹ as defined above, under standard amide bond forming conditions using a coupling reagent (e.g. HOBT, EDC and DIPEA in dry DMF) affording amide (III), wherein R² and R¹ are as defined above. Amines (II) are used as single isomers or as mixtures of two isomers; therefore amides (III) are isolated as mixtures of two isomers or as single isomers.

### General method B:

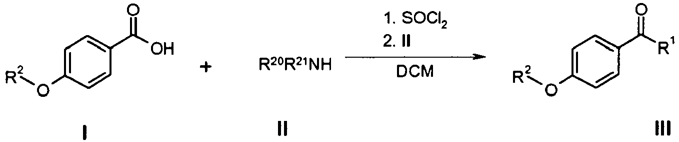

By allowing an acid (I), wherein R² is as defined as above, to form the corresponding acid chloride by reaction with thionyl chloride, and then reacting the acid chloride with an amine (II), wherein R²⁰ and R²¹ together with the amine that the groups are attached to form R¹ as defined above, under basic conditions (e.g. triethyl amine, DIPEA, K₂CO₃ and the like) in a solvent (DCM, DMF, THF, NMP and the like) affording amide (III), wherein R² and R¹ are as defined above. Amines (II) are used as single isomers or as mixtures of two isomers; therefore amides (III) are isolated as mixtures of two isomers or as single isomers.

Alternatively, the acid chloride corresponding to acid (I), wherein R² is as defined above, can be used directly to react with amine (II), wherein R²⁰ and R²¹ together with the amine that the groups are attached to form R¹ as defined above, under similar basic conditions.

### General method C:

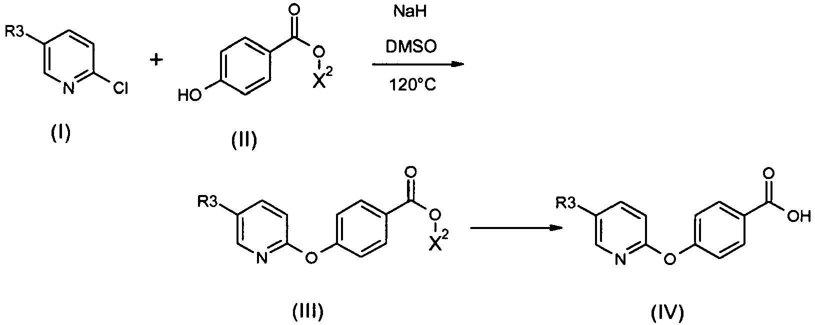

The phenyl ester (III), wherein R² is as defined above, can be syntheisized as described below or by other procedures known by people skilled in the art or described in the literature. C(O)OX² forms a suitable ester. (II) was dissolved in dry DMSO at RT under N₂. NaH was added slowly. After the deprotonation has taken place, (I) was added and the reaction mixture was heated to 120°C for 10-20 h. The reaction can be followed by LC-MS. The reaction mixture was poored on ice-water and extracted with and organic solvent to give (III). The ester was hydrolysed under basic conditions to give (IV), wherein R² is as defined above.

Compounds shown in the below table can be synthesized by the general methods listed above and by suitable methods known for people skilled in the art or as described in the litterature.

| **No** | **Molecule** | **Name** | **LC-MS (electrospray)** |
|---|---|---|---|
| 1 | 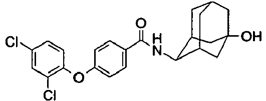 | 4-(2,4-Dichloro-phenoxy)-N-(5-hydroxy-adamantan-2-yl)-benzamide | Rt= 2,06 min m/z: 432 M+1 |
| 2 | 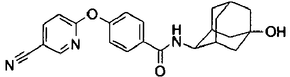 | 4-(5-Cyano-pyridin-2-yloxy)-N-(5-hydroxy-adamantan-2-yl)-benzamide | Rt= 1,48 min m/z: 391 M+1 |
| 3 | 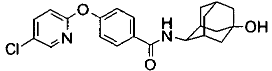 | 4-(5-Chloro-pyridin-2-yloxy)-N-(5-hydroxy-adamantan-2- | Rt= 1,72 min m/z: 399 M+1 |
| | | yl)-benzamide | |
| 4 | 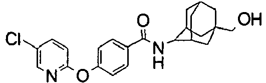 | 4-(5-Chloro-pyridin-2-yloxy)-N-(5-hydroxymethyl-adamantan-2-yl)-benzamide | Rt= 1,87 min. m/z: 413 M+1 |
| 5 | 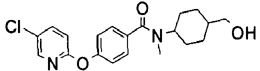 | 4-(5-Chloro-pyridin-2-yloxy)-N-(4-hydroxymethyl-cyclohexyl)-N-methyl-benzamide | Rt= 1,60 min. m/z: 376 M+1 |
| 6 | 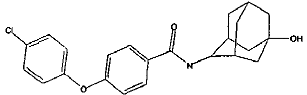 | 4-(4-Chloro-phenoxy)-N-(5-hydroxy-adamantan-2-yl)-benzamide | Rt= 1,49 mn. M+1 : 397.8 |
| 7 | 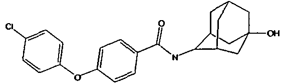 | 4-(4-Chloro-phenoxy)-N-(5-hydroxy-adamantan-2-yl)-benzamide | Rt = 1,47 min. M+1 : 397.8 |
| 8* | 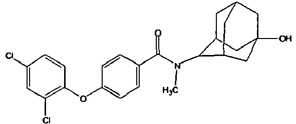 | 4-(2,4-Dichloro-phenoxy)-N-(5-hydroxy-adamantan-2-yl)-N-methyl-benzamide | Rt= 2,08 min. M+1 : 446 |
| 9* | 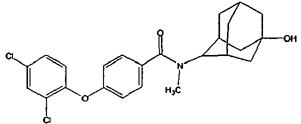 | 4-(2,4-Dichloro-phenoxy)-N-(5-hydroxy-adamantan-2-yl)-N-methyl-benzamide | Rt= 2,15 min. M+ 1 : 446 |
| 10 | 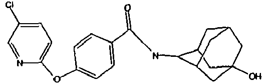 | 4-(5-Chloro-pyridin-2-yloxy)-N-(5-hydroxy-adamantan-2-yl)-benzamide | Rt = 1,13 min. M+1 : 399.1 |
| 11 | 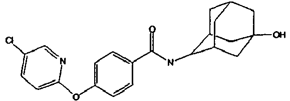 | 4-(5-Chloro-pyridin-2-yloxy)-N-(5-hydroxy-adamantan-2-yl)-benzamide | Rt = 1,18 min. M+1 : 399.1 |

| | | | |
|---|---|---|---|
| * = comparative example | | | |

### PHARMACOLOGICAL METHODS

### 11βHSD1 enzyme assay

### Materials

³H-cortisone and anti-rabbit Ig coated scintillation proximity assay (SPA) beads were purchased from Amersham Pharmacia Biotech, β-NADPH was from Sigma and rabbit anti-cortisol antibodies were from Fitzgerald. An extract of yeast transformed with h-11βHSD1 (Hult et al., FEBS Lett., 441, 25 (1998)) was used as the source of enzyme. The test compounds were dissolved in DMSO (10 mM). All dilutions were performed in a buffer containing 50 mM TRIS-HCl (Sigma Chemical Co), 4 mM EDTA (Sigma Chemical Co), 0.1% BSA (Sigma Chemical Co), 0.01% Tween-20 (Sigma Chemical Co) and 0.005% bacitracin (Novo Nordisk A/S), pH=7.4. Optiplate 96 wells plates were supplied by Packard. The amount of ³H-cortisol bound to the SPA beads was measured on TopCount NXT, Packard.

### Methods

h-11βHSD1, 120 nM ³H-cortisone, 4 mM β-NADPH, antibody (1:200), serial dilutions of test compound and SPA particles (2 mg/well) were added to the wells. The reaction was initiated by mixing the different components and was allowed to proceed under shaking for 60 min at 30°C. The reaction was stopped be the addition of 10 fold excess of a stopping buffer containing 500 µM carbenoxolone and 1 µM cortisone. Data was analysed using GraphPad Prism software.

**Table 1**

| Inhibition of h-11βHSD1 by compounds of the invention | |
|---|---|
| **No** | **h-11βHSD1 IC50 (nM)** |
| 1 | 73; 81 |
| 2 | 259; 305 |
| 3 | 56; 89 |
| 4 | 51; 56; 51 |
| 5 | 17; 27 |

Preferred features of the invention:
1. A compound of the general formula (I): 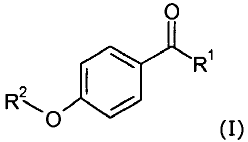 wherein R¹ is selected from the group consisting of: 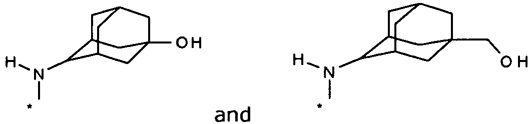
   wherein the symbol * denotes the point of attachment,
   R² is selected from the group consisting of phenyl substituted with one or two independently selected R³ and pyridinyl substituted with one or two independently selected R³;
   R³ is selected from the group consisting of halogen, cyano, -C(=O)OH, -C(=O)R⁴, -CH(OH)R⁴, C(=O)-NR⁶R⁷, -OR⁴, -SR⁴, -S(=O)₂R⁴, -S(=O)₂-NR⁶R⁷, -C=CR⁴R⁵, -C≡C-R⁴, -C₃-C₁₀heterocyclyl optionally substituted with halogen or methyl, C₃-C₁₀cycloalkyl optionally substituted with halogen, methyl or hydroxy, phenyl optionally substituted with -C(=O)OH, halogen or methyl, C₁-C₆alkyl optionally substituted with R⁴ and heteroaryl optionally substituted with -C(=O)OH, halogen or methyl;
   R⁴ is selected from the group consisting of hydrogen, C₁-C₆alkyl, phenyl, heteroaryl and C₃-C₁₀cycloalkyl, wherein said C₁-C₆alkyl, phenyl, heteroaryl and C₃-C₁₀cycloalkyl are optionally substituted with -C(=O)OH, -CH₂OH, halogen, methyl, triflouromethyl, methoxy or hydroxyl and -C(=O)NH₂;
   R⁵ is selected from the group consisting of hydrogen, C₁-C₆alkyl, phenyl, heteroaryl and C₃-C₁₀cycloalkyl, wherein said C₁-C₆alkyl, phenyl, heteroaryl and C₃-C₁₀cycloalkyl are optionally substituted with -C(=O)OH, -CH₂OH, halogen, methyl, triflouromethyl, methoxy or hydroxy;
   R⁶ is selected from the group consisting of hydrogen, C₁-C₆alkyl, tetrahydropyranyl and C₃-C₁₀cycloalkyl, wherein said C₁-C₆alkyl, tetrahydropyranyl, and C₃-C₁₀cycloalkyl are optionally substituted with one or two substituents independently selected from the group consisting of halogen and hydroxy;
   R⁷ is selected from the group consisting of hydrogen, C₁-C₆alkyl, tetrahydropyranyl and C₃-C₁₀cycloalkyl, wherein said C₁-C₆alkyl, tetrahydropyranyl, and C₃-C₁₀cycloalkyl are optionally substituted with one or two substituents independently selected from the group consisting of halogen and hydroxy;
   or R⁶ and R⁷ together with the nitrogen atom to which they are attached form a piperidine or a pyrrolidine ring, wherein said ring is optionally substituted with hydroxy or halogen;
   or a salt thereof with a pharmaceutically acceptable acid or base, or any optical isomer or mixture of optical isomers, including a racemic mixture, or any tautomeric forms.
2. The compound according to clause 1, wherein R¹ is 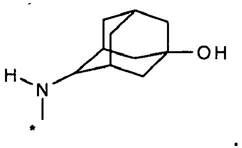
3. The compound according to clause 1, wherein R¹ is 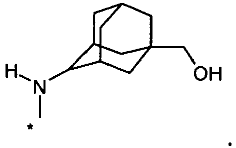
4. The compound according to any one of the clauses 1-3, wherein R² is phenyl substituted with one or two independently selected R³.
5. The compound according to any one of the clauses 1-3, wherein R² is pyridinyl substituted with one or two independently selected R³.
6. The compound according to any one of the clauses 1-5, wherein R² is substituted with one R³.
7. The compound according to any one of the clauses 1-5, wherein R² is substituted with two independently selected R³.
8. The compound according to any one of the clauses 1-7, wherein R³ is selected from the group consisting of cyano and halogen.
9. The compound according to any one of the clauses 1-8, wherein R² is substituted with one R³, which is cyano.
10. The compound according to any one of the clauses 1-8, wherein R² is substituted with two R³, which are each halogen.
11. The compound according to any one of the clauses 1-8 and 10, wherein R³ is chlorine.
12. The compound according to any one of the clauses 1-11, wherein R⁴ is selected from the group consisting of hydrogen, methyl, ethyl, phenyl, pyridinyl, imidazolyl, cyclopropyl, cyclobutyl and cyclohexyl, wherein said methyl, ethyl, phenyl, pyridinyl, imidazolyl, cyclopropyl, cyclobutyl and cyclohexyl are optionally substituted with -C(=O)OH, -CH₂OH, chlorine, fluorine, methyl, triflouromethyl, methoxy or hydroxy.
13. The compound according to any one of the clauses 1-12, wherein R⁵ is selected from the group consisting of hydrogen, methyl, ethyl, phenyl, pyridinyl, imidazolyl, cyclopropyl, cyclobutyl and cyclohexyl, wherein said methyl, ethyl, phenyl, pyridinyl, imidazolyl, cyclopropyl, cyclobutyl and cyclohexyl are optionally substituted with -C(=O)OH, -CH₂OH, chlorine, fluorine, methyl, triflouromethyl, methoxy or hydroxy.
14. The compound according to any one of the clauses 1-13, wherein R⁶ is selected from the group consisting of hydrogen, methyl, ethyl, tetrahydropyranyl, cyclopropyl, cyclobutyl and cyclohexyl, wherein said methyl, ethyl, tetrahydropyranyl, cyclopropyl, cyclobutyl, and cyclohexyl are optionally substituted with one or two substituents independently selected from the group consisting of chlorine, fluorine and hydroxy.
15. The compound according to any one of the clauses 1-14, wherein R⁷ is selected from the group consisting of hydrogen, methyl, ethyl, tetrahydropyranyl, cyclopropyl, cyclobutyl and cyclohexyl, wherein said methyl, ethyl, tetrahydropyranyl, cyclopropyl, cyclobutyl, and cyclohexyl are optionally substituted with one or two substituents independently selected from the group consisting of chlorine, fluorine and hydroxy.
16. The compound according to any one of the clauses 1-13, wherein R⁶ and R⁷ together with the nitrogen atom to which they are attached form a piperidine or a pyrrolidine ring, wherein said ring is optionally substituted with hydroxy, chlorine or fluorine.
17. The compound according to any one of the clauses 1-16, wherein heteroaryl is selected from the group consisting of isoxazolyl, pyridazinyl, pyrimidinyl, imidazolyl, pyrazolyl and pyridinyl.
18. The compound according to any one of the clauses 1-16, wherein C₃-C₁₀cycloalkyl is selected from the group consisting of cyclopropyl, cyclobutyl, cyclopentyl and cyclohexyl.
19. The compound according to any one of the clauses 1-16, wherein C₃-C₁₀heterocyclyl is selected from the group consisting of pyrrolidinyl, tetrahydropyranyl, piperidinyl, piperazinyl, and morpholinyl.
20. The compound selected from the group consisting of 4-(2,4-Dichloro-phenoxy)-N-(5-hydroxy-adamantan-2-yl)-benzamide, 4-(5-Cyano-pyridin-2-yloxy)-N-(5-hydroxy-adamantan-2-yl)-benzamide, 4-(5-Chloro-pyridin-2-yloxy)-N-(5-hydroxy-adamantan-2-yl)-benzamide, 4-(5-Chloro-pyridin-2-yloxy)-N-(5-hydroxymethyl-adamantan-2-yl)-benzamide, and 4-(5-Chloro-pyridin-2-yloxy)-N-(4-hydroxymethyl-cyclohexyl)-N-methyl-benzamide.
21. A compound according to any one of the above clauses, which is an agent useful for the treatment, prevention and/or prophylaxis of any conditions, disorders and diseases wherein a modulation or an inhibition of the activity of 11βHSD1 is beneficial.
22. A compound according to any one of the clauses 1-20, which is an agent useful for the treatment, prevention and/or prophylaxis of any conditions, disorders and diseases that are influenced by intracellular glucocorticoid levels.
23. A compound according to any one of the clauses 1-20, which is an agent useful for the treatment, prevention and/or prophylaxis of conditions, disorders or diseases selected from the group consisting of the metabolic syndrome, insulin resistance, dyslipidemia, hypertension and obesity.
24. A compound according to any one of the clauses 1-20, which is an agent useful for the treatment, prevention and/or prophylaxis of type 2 diabetes, impaired glucose tolerance (IGT), impaired fasting glucose (IFG).
25. A compound according to any one of the clauses 1-20, which is an agent useful for the delaying or prevention of the progression from IGT into type 2 diabetes.
26. A compound according to any one of the clauses 1-20, which is an agent useful for delaying or prevention of the progression of the metabolic syndrome into type 2 diabetes.
27. A compound according to any one of the clauses 1-20, which is an agent useful for the treatment, prevention and/or prophylaxis of adverse effects of glucocorticoid receptor agonist treatment or therapy.
28. A pharmaceutical composition comprising, as an active ingredient, at least one compound according to any one of the clauses 1-20 together with one ore more pharmaceutically acceptable carriers or excipients.
29. The pharmaceutical composition according to clause 28 which is for oral, nasal, buccal, transdermal, pulmonal or parenteral administration.
30. The pharmaceutical composition according to clause 28 or 29 in unit dosage form, comprising from 0.05 mg to 2000 mg/day, from 0.1 mg to 1000 mg or from 0.5 mg to 500
31. A compound according to any one of the clauses 1-20 for use in a method for the treatment, prevention and/or prophylaxis of any conditions, disorders or diseases wherein a modulation or an inhibition of the activity of 11βHSD1 is beneficial, the method comprising administering to a subject in need thereof an effective amount of a compound according to any of the clauses 1-22.
32. A compound for use according to clause 31 for use in a method according to clause 31 wherein the conditions, disorders or diseases are selected from the group consisting of the metabolic syndrome, insulin resistance, dyslipidemia, hypertension and obesity.

## Claims

1. A compound of the general formula (I): 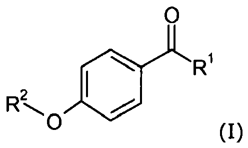 wherein R¹ is selected from the group consisting of: 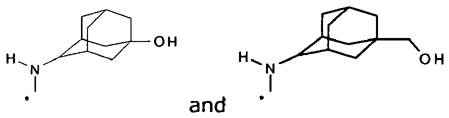
wherein the symbol * denotes the point of attachment,
R² is selected from the group consisting of phenyl substituted with one or two independently selected R³ and pyridinyl substituted with one or two independently selected R³;
R³ is selected from the group consisting of halogen, cyano, -C(=O)OH, -C(=O)R⁴, -CH(OH)R⁴, C(=O)-NR⁶R⁷, -OR⁴, -SR⁴, -S(=O)₂R⁴, -S(=O)₂-NR⁶R⁷, -C=CR⁴R⁵, -C≡C-R⁴, -C₃-C₁₀heterocyclyl optionally substituted with halogen or methyl, a saturated carbocyclic ring having from 3 to 10 carbon atoms optionally containing one or two carbon bridges wherein the carbocyclic ring is optionally substituted with halogen, methyl or hydroxy, phenyl optionally substituted with -C(=O)OH, halogen or methyl, C₁-C₆alkyl optionally substituted with R⁴ and heteroaryl optionally substituted with -C(=O)OH, halogen or methyl;
R⁴ is selected from the group consisting of hydrogen, C₁-C₆alkyl, phenyl, heteroaryl and a saturated carbocyclic ring having from 3 to 10 carbon atoms optionally containing one or two carbon bridges, wherein said C₁-C₆alkyl, phenyl, heteroaryl and saturated carbocyclic ring are optionally substituted with -C(=O)OH, -CH₂OH, halogen, methyl, triflouromethyl, methoxy or hydroxyl and -C(=O)NH₂;
R⁵ is selected from the group consisting of hydrogen, C₁-C₆alkyl, phenyl, heteroaryl anda saturated carbocyclic ring having from 3 to 10 carbon atoms optionally containing one or two carbon bridges, wherein said C₁-C₆alkyl, phenyl, heteroaryl and carbocyclic ring are optionally substituted with -C(=O)OH, -CH₂OH, halogen, methyl, triflouromethyl, methoxy or hydroxy;
R⁶ is selected from the group consisting of hydrogen, C₁-C₆alkyl, tetrahydropyranyl and a saturated carbocyclic ring having from 3 to 10 carbon atoms optionally containing one or two carbon bridges, wherein said C₁-C₆alkyl, tetrahydropyranyl, and carbocyclic ring are optionally substituted with one or two substituents independently selected from the group consisting of halogen and hydroxy;
R⁷ is selected from the group consisting of hydrogen, C₁-C₆alkyl, tetrahydropyranyl and a saturated carbocyclic ring having from 3 to 10 carbon atoms optionally containing one or two carbon bridges, wherein said C₁-C₆alkyl, tetrahydropyranyl, and carbocyclic ring are optionally substituted with one or two substituents independently selected from the group consisting of halogen and hydroxy;
or R⁶ and R⁷ together with the nitrogen atom to which they are attached form a piperidine or a pyrrolidine ring, wherein said ring is optionally substituted with hydroxy or halogen;
or a salt thereof with a pharmaceutically acceptable acid or base, or any optical isomer or mixture of optical isomers, including a racemic mixture, or any tautomeric forms.

2. The compound according to claim 1, wherein R¹ is

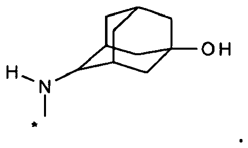

3. The compound according to claim 1, wherein R¹ is

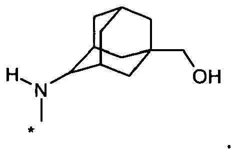

4. The compound according to any one of the claims 1 to 3, wherein R² is phenyl substituted with one or two substituents independently selected from R³.

5. The compound according to any one of the claims 1 to 3, wherein R² is pyridinyl substituted with one or two substituents independently selected from R³.

6. The compound according to any one of the claims 1 to 5, wherein R² is substituted with one substituent selected from R³.

7. The compound according to any one of the claims 1 to 5, wherein R² is substituted with two substituents independently selected from R³.

8. The compound according to any one of the claims 1 to 7, wherein R³ is chlorine.

9. The compound of claim 1, wherein the compound is 4-(5-Chloro-pyridin-2-yloxy)-N-(5-hydroxy-adamantan-2-yl)-benzamide or a pharmaceutically acceptable salt thereof.

10. The compound of claim 1, wherein the compound is 4-(5-Chloro-pyridin-2-yloxy)-N-(5-hydroxymethyl-adamantan-2-yl)-benzamide or a pharmaceutically acceptable salt thereof.

11. A combination of a compound as defined in any one of claims 1 to 10 and one or more further active substances.

12. A pharmaceutical composition comprising, as an active ingredient, at least one compound according to any one of the claims 1-10, or a combination according to claim 11, together with one or more pharmaceutically acceptable carriers or excipients.

13. A compound as defined in any one of claims 1 to 10, a combination as defined in claim 11, or a composition as defined in claim 12, for use as a medicament.

14. A compound, combination or composition according to claim 13 for use in the treatment, prevention and/or prophylaxis of any conditions, disorders and diseases wherein a modulation or an inhibition of the activity of 11βHSD1 is beneficial; or the treatment, prevention and/or prophylaxis of any conditions, disorders and diseases that are influenced by intracellular glucocorticoid levels.

15. A compound, combination or composition according to claim 13 for use in the treatment, prevention and/or prophylaxis of conditions, disorders or diseases selected from the group consisting of the metabolic syndrome, insulin resistance, dyslipidemia, hypertension, obesity, type 2 diabetes, impaired glucose tolerance (IGT), impaired fasting glucose (IFG), or the treatment, prevention and/or prophylaxis of adverse effects of glucocorticoid receptor agonist treatment or therapy.

## Patentansprüche

1. Verbindung der allgemeinen Formel (I): 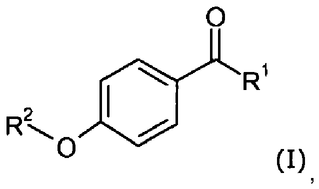 wobei R¹ aus der Gruppe bestehend aus: 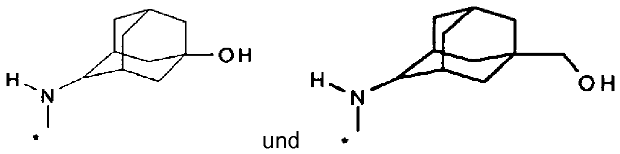
ausgewählt ist, wobei das Symbol * die Anlagerungsstelle kennzeichnet,
R² aus der Gruppe bestehend aus Phenyl, das mit einem oder zwei unabhängig ausgewählten R³ substituiert ist, und Pyridinyl, das mit einem oder zwei unabhängig ausgewählten R³ substituiert ist, ausgewählt ist;
R³ aus der Gruppe bestehend aus Halogen, Cyano, -C(=O)OH, -C(=O)R⁴, -CH(OH)R⁴, C(=O)-NR⁶R⁷, -OR⁴, -SR⁴, -S(=O)₂R⁴, -S(=O)₂-NR⁶R⁷, -C=CR⁴R⁵, -C≡C-R⁴, -C₃-C₁₀-Heterocyclyl, das gegebenenfalls mit Halogen oder Methyl substituiert ist, einem gesättigten carbocyclischen Ring mit 3 bis 10 Kohlenstoffatomen, der gegebenenfalls ein oder zwei Kohlenstoffbrücken enthält, wobei der carbocyclische Ring gegebenenfalls mit Halogen, Methyl oder Hydroxy substituiert ist, Phenyl, das gegebenenfalls mit -C(=O)OH, Halogen oder Methyl substituiert ist, C₁-C₆-Alkyl, das gegebenenfalls mit R⁴ substituiert ist, und Heteroaryl, das gegebenenfalls mit -C(=O)OH, Halogen oder Methyl substituiert ist, ausgewählt ist;
R⁴ aus der Gruppe bestehend aus Wasserstoff, C₁-C₆-Alkyl, Phenyl, Heteroaryl und einem gesättigten carbocyclischen Ring mit 3 bis 10 Kohlenstoffatomen, der gegebenenfalls ein oder zwei Kohlenstoffbrücken enthält, wobei das C₁-C₆-Alkyl, das Phenyl, das Heteroaryl und der gesättigte carbocyclische Ring gegebenenfalls mit -C(=O)OH, -CH₂OH, Halogen, Methyl, Trifluormethyl, Methoxy oder Hydroxyl und -C(=O)NH₂ substituiert sind, ausgewählt ist;
R⁵ aus der Gruppe bestehend aus Wasserstoff, C₁-C₆-Alkyl, Phenyl, Heteroaryl und einem gesättigten carbocyclischen Ring mit 3 bis 10 Kohlenstoffatomen, der gegebenenfalls ein oder zwei Kohlenstoffbrücken enthält, wobei das C₁-C₆-Alkyl, das Phenyl, das Heteroaryl und der carbocyclische Ring gegebenenfalls mit -C(=O)OH, -CH₂OH, Halogen, Methyl, Trifluormethyl, Methoxy oder Hydroxy substituiert sind, ausgewählt ist;
R⁶ aus der Gruppe bestehend aus Wasserstoff, C₁-C₆-Alkyl, Tetrahydropyranyl und einem gesättigten carbocyclischen Ring mit 3 bis 10 Kohlenstoffatomen, der gegebenenfalls ein oder zwei Kohlenstoffbrücken enthält, wobei das C₁-C₆-Alkyl, das Tetrahydropyranyl und der carbocyclische Ring gegebenenfalls mit einem oder zwei Substituenten substituiert sind, die unabhängig aus der Gruppe bestehend aus Halogen und Hydroxy ausgewählt sind, ausgewählt ist;
R⁷ aus der Gruppe bestehend aus Wasserstoff, C₁-C₆-Alkyl, Tetrahydropyranyl und einem gesättigten carbocyclischen Ring mit 3 bis 10 Kohlenstoffatomen, der gegebenenfalls ein oder zwei Kohlenstoffbrücken enthält, wobei das C₁-C₆-Alkyl, das Tetrahydropyranyl und der carbocyclische Ring gegebenenfalls mit einem oder zwei Substituenten substituiert sind, die unabhängig aus der Gruppe bestehend aus Halogen und Hydroxy ausgewählt sind, ausgewählt ist;
oder R⁶ und R⁷ zusammen mit dem Stickstoffatom, an das sie angelagert sind, einen Piperidinring oder einen Pyrrolidinring bilden, wobei der Ring gegebenenfalls mit Hydroxy oder Halogen substituiert ist;
oder ein Salz davon mit einer pharmazeutisch unbedenklichen Säure oder Base oder ein beliebiges optisches Isomer oder eine beliebige Mischung von optischen Isomeren, einschließlich einer racemischen Mischung, oder beliebige tautomere Formen.

2. Verbindung nach Anspruch 1, wobei R¹ 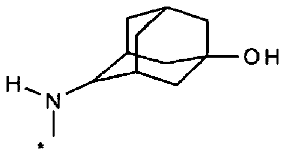 ist.

3. Verbindung nach Anspruch 1, wobei R¹ 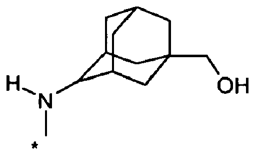 ist.

4. Verbindung nach einem der Ansprüche 1 bis 3, wobei R² Phenyl ist, das mit einem oder zwei Substituenten substituiert ist, die unabhängig aus R³ ausgewählt sind.

5. Verbindung nach einem der Ansprüche 1 bis 3, wobei R² Pyridinyl ist, das mit einem oder zwei Substituenten substituiert ist, die unabhängig aus R³ ausgewählt sind.

6. Verbindung nach einem der Ansprüche 1 bis 5, wobei R² mit einem Substituenten substituiert ist, der aus R³ ausgewählt ist.

7. Verbindung nach einem der Ansprüche 1 bis 5, wobei R² mit zwei Substituenten substituiert ist, die unabhängig aus R³ ausgewählt sind.

8. Verbindung nach einem der Ansprüche 1 bis 7, wobei R³ Chlor ist.

9. Verbindung nach Anspruch 1, wobei die Verbindung 4-(5-Chlorpyridin-2-yloxy)-N-(5-hydroxyadamantan-2-yl)benzamid oder ein pharmazeutisch unbedenkliches Salz davon ist.

10. Verbindung nach Anspruch 1, wobei die Verbindung 4-(5-Chlorpyridin-2-yloxy)-N-(5-hydroxymethyladamantan-2-yl)benzamid oder ein pharmazeutisch unbedenkliches Salz davon ist.

11. Kombination einer Verbindung nach einem der Ansprüche 1 bis 10 und einer oder mehrerer weiteren Wirksubstanzen.

12. Pharmazeutische Zusammensetzung, die als einen Wirkbestandteil mindestens eine Verbindung nach einem der Ansprüche 1 - 10 oder eine Kombination nach Anspruch 11 zusammen mit einem oder mehreren pharmazeutisch unbedenklichen Trägerstoffen oder Hilfsstoffen umfasst.

13. Verbindung nach einem der Ansprüche 1 bis 10, Kombination nach Anspruch 11 oder Zusammensetzung nach Anspruch 12 zur Verwendung als ein Arzneimittel.

14. Verbindung, Kombination oder Zusammensetzung nach Anspruch 13 zur Verwendung bei der Behandlung, Prävention und/oder Prophylaxe von beliebigen Leiden, Erkrankungen oder Krankheiten, bei denen eine Modulation oder eine Hemmung der Aktivität von 11βHSD1 zuträglich ist; oder bei der Behandlung, Prävention und/oder Prophylaxe von beliebigen Leiden, Erkrankungen oder Krankheiten, die von intrazellulären Glukokortikoidspiegeln beeinflusst werden.

15. Verbindung, Kombination oder Zusammensetzung nach Anspruch 13 zur Verwendung bei der Behandlung, Prävention und/oder Prophylaxe von Leiden, Erkrankungen oder Krankheiten, die aus der Gruppe bestehend aus metabolischem Syndrom, Insulinresistenz, Dyslipidämie, Hypertonie, Adipositas, Typ-2-Diabetes, gestörter Glukosetoleranz (IGT), gestörter Nüchternglukose (IFG) ausgewählt sind, oder bei der Behandlung, Prävention und/oder Prophylaxe von Nebenwirkungen von einer Behandlung oder Therapie mit Glukokortikoid-Rezeptoragonist.

## Revendications

1. Composé de formule générale (I) : 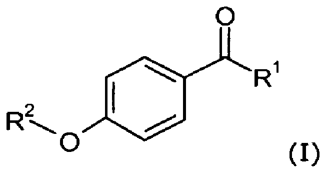 dans lequel R¹ est choisi dans le groupe constitué par : 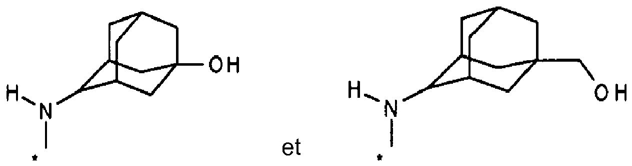
dans lequel le symbole * désigne le point de liaison,
R² est choisi dans le groupe constitué par le phényle substitué par un ou deux R³ choisis indépendamment et par le pyridinyle substitué par un ou deux R³ choisis indépendamment ; R³ est choisi dans le groupe constitué par l'halogène, le cyano, -C(=O)OH, -C(=O)R⁴, - CH(OH)R⁴, C(=O)-NR⁶R⁷, -OR⁴, -SR⁴, -S(=O)₂R⁴, -S(=O)₂-NR⁶R⁷, -C=CR⁴R⁵, -C=C-R⁴, l'hétérocyclyle en C₃-C₁₀ facultativement substitué par de l'halogène ou du méthyle, un cycle carbocyclique saturé ayant entre 3 et 10 atomes de carbone contenant facultativement une ou deux liaisons carbone dans lequel le cycle carbocyclique est facultativement substitué par de l'halogène, du méthyle ou de l'hydroxy, le phényle facultativement substitué par -C(=O)OH, de l'halogène ou du méthyle, l'alkyle en C₁-C₆ facultativement substitué par R⁴ et l'hétéroaryle facultativement substitué par -C(=O)OH, de l'halogène ou du méthyle ;
R⁴ est choisi dans le groupe constitué par l'hydrogène, l'alkyle en C₁-C₆, le phényle, l'hétéroaryle et un cycle carbocyclique saturé ayant entre 3 et 10 atomes de carbone contenant facultativement une ou deux liaisons carbone, dans lequel lesdits alkyle en C₁-C₆, phényle, hétéroaryle et cycle carbocyclique saturé sont facultativement substitués par -C(=O)OH, -CH₂OH, de l'halogène, du méthyle, du trifluorométhyle, du méthoxy ou de l'hydroxyle et -C(=O)NH₂ ;
R⁵ est choisi dans le groupe constitué par l'hydrogène, l'alkyle en C₁-C₆, le phényle, l'hétéroaryle et un cycle carbocyclique saturé ayant entre 3 et 10 atomes de carbone contenant facultativement une ou deux liaisons carbone, dans lequel lesdits alkyle en C₁-C₆, phényle, hétéroaryle et cycle carbocyclique sont facultativement substitués par -C(=O)OH, - CH₂OH, de l'halogène, du méthyle, du trifluorométhyle, du méthoxy ou de l'hydroxy ;
R⁶ est choisi dans le groupe constitué par l'hydrogène, l'alkyle en C₁-C₆, le tétrahydropyranyle et un cycle carbocyclique saturé ayant entre 3 et 10 atomes de carbone contenant facultativement une ou deux liaisons carbone, dans lequel lesdits alkyle en C₁-C₆, tétrahydropyranyle et cycle carbocyclique sont facultativement substitués par un ou deux substituants indépendamment choisis dans le groupe constitué par l'halogène et l'hydroxy ;
R⁷ est choisi dans le groupe constitué par l'hydrogène, l'alkyle en C₁-C₆, le tétrahydropyranyle et un cycle carbocyclique saturé ayant entre 3 et 10 atomes de carbone contenant facultativement une ou deux liaisons carbone, dans lequel lesdits alkyle en C₁-C₆, tétrahydropyranyle et cycle carbocyclique sont facultativement substitués par un ou deux substituants indépendamment choisis dans le groupe constitué par l'halogène et l'hydroxy ; ou R⁶ et R⁷, conjointement avec l'atome d'azote auquel ils sont liés, forment un cycle pipéridine ou pyrrolidine, dans lequel ledit cycle est facultativement substitué par de l'hydroxy ou de l'halogène ;
ou sel de celui-ci avec un acide ou une base pharmaceutiquement acceptable, ou tout isomère optique ou mélange d'isomères optiques, notamment un mélange racémique, ou toute forme tautomère.

2. Composé selon la revendication 1, dans lequel R¹ est

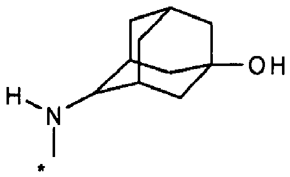

3. Composé selon la revendication 1, dans lequel R¹ est

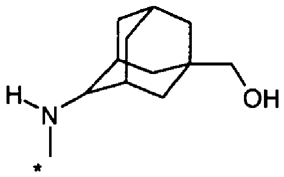

4. Composé selon l'une quelconque des revendications 1 à 3, dans lequel R² est du phényle substitué par un ou deux substituants indépendamment choisis parmi R³.

5. Composé selon l'une quelconque des revendications 1 à 3, dans lequel R² est du pyridinyle substitué par un ou deux substituants indépendamment choisis parmi R³.

6. Composé selon l'une quelconque des revendications 1 à 5, dans lequel R² est substitué par un substituant choisi parmi R³.

7. Composé selon l'une quelconque des revendications 1 à 5, dans lequel R² est substitué par deux substituants indépendamment choisis parmi R³.

8. Composé selon l'une quelconque des revendications 1 à 7, dans lequel R³ est du chlore.

9. Composé selon la revendication 1, dans lequel le composé est du 4-(5-Chloro-pyridin-2-yloxy)-N-(5-hydroxy-adamantan-2-yl)-benzamide ou un sel pharmaceutiquement acceptable de celui-ci.

10. Composé selon la revendication 1, dans lequel le composé est du 4-(5-Chloro-pyridin-2-yloxy)-N-(5-hydroxyméthyl-adamantan-2-yl)-benzamide ou un sel pharmaceutiquement acceptable de celui-ci.

11. Combinaison d'un composé selon l'une quelconque des revendications 1 à 10 et d'un ou de plusieurs autres principes actifs.

12. Composition pharmaceutique comprenant, comme principe actif, au moins un composé selon l'une quelconque des revendications 1 à 10, ou une combinaison selon la revendication 11, conjointement avec un ou plusieurs supports ou excipients pharmaceutiquement acceptables.

13. Composé selon l'une quelconque des revendications 1 à 10, combinaison selon la revendication 11 ou composition selon la revendication 12, pour une utilisation en tant que médicament.

14. Composé, combinaison ou composition selon la revendication 13 pour une utilisation dans le traitement, la prévention et/ou la prophylaxie de toute condition, trouble et maladie dans lequel/laquelle une modulation ou une inhibition de l'activité de 11βHSD1 est bénéfique ; ou dans le traitement, la prévention et/ou la prophylaxie de toute condition, trouble et maladie influencé(e) par des taux de glucocorticoïdes intracellulaires.

15. Composé, combinaison ou composition selon la revendication 13 pour une utilisation dans le traitement, la prévention et/ou la prophylaxie de conditions, troubles ou maladies choisis dans le groupe constitué par le syndrome métabolique, l'insulinorésistance, la dyslipidémie, l'hypertension, l'obésité, le diabète de type 2, l'intolérance au glucose (IGT), l'hyperglycémie à jeun (IFG) ou dans le traitement, la prévention et/ou la prophylaxie d'effets indésirables liés au traitement ou à la thérapie par l'agoniste du récepteur de glucocorticoïdes.
